# EUROPEAN PATENT SPECIFICATION

(11) **EP 2 279 227 B1**
(45) Date of publication and mention of the grant of the patent: **14.08.2013**
(21) Application number: 09806933.9
(22) Date of filing: 04.05.2009
(51) Int. Cl.: A61K 9/16, C08J 3/05, C08F 2/26

(54) **ANIONIC LATEX AS A CARRIER FOR ACTIVE INGREDIENTS AND METHODS FOR MAKING AND USING THE SAME**
ANIONISCHES LATEX ALS TRÄGER FÜR WIRKSTOFFE SOWIE VERFAHREN ZU SEINER HERSTELLUNG UND VERWENDUNG
LATEX ANIONIQUE UTILISÉ COMME VÉHICULE D'INGRÉDIENTS ACTIFS ET PROCÉDÉS DE FABRICATION ET D'UTILISATION DE CE DERNIER

(30) Priority: 06.05.2008 US 116040
(43) Date of publication of application: 02.02.2011
(73) Proprietor: Mallard Creek Polymers, Inc, Charlotte, NC 28262 (US)
(72) Inventor: KRISHNAN, Venkataram, Cary NC 26158 (US)
(74) Representative: Gillard, Richard Edward
(86) International application number: PCT/US2009/002740
(87) International publication number: WO 2010/019180

(56) References cited:
- WO-A2-2008/024509
- WO-A2-2008/088394
- US-A- 4 902 503
- US-A- 5 629 365
- US-A- 5 788 687
- US-A1- 2003 125 453
- US-A1- 2008 057 049
- US-A1- 2008 171 804

## Description

### FIELD OF THE INVENTION

This invention relates to the field of polymeric materials that can be used in combination with a wide variety of substrates, such as personal care products, textiles, metal, cellulosic materials, plastics, and the like. This invention further relates to latex polymer coatings that comprise at least one active component as well as methods for making and using such latex compositions.

### BACKGROUND OF THE INVENTION

WO 2008/088394 is an Article 54(3) EPC prior art document to the present invention and discloses the subject-matter of the disclaimer of the claims. WO 2008/088394 discloses latex compositions that incorporate at least one bioactive component such as an antibacterial or an antifungal agent, and methods for making and using such latex compositions. The latex compositions disclosed therein can be prepared by the emulsion polymerization of the latex component monomers in the presence of the at least one bioactive component.

WO 2008/024509 discloses cationic latex compositions that incorporate at least one bioactive component such as an antibacterial or antifungal agent, and methods for making and using such latex compositions. The latex compositions disclosed therein can be prepared by the emulsion polymerization of the latex component monomers in the presence of the at least one bioactive component.

The deposition of latex polymer coatings on solid substrates has long been utilized to impart certain end-use performance properties to those substrates, such as hydrophobicity, strength, adhesive properties, compatibility, and the like. Depending upon the selection of the starting monomers, surfactants, emulsion polymerization conditions, and other parameters, the deposited polymers can be designed to carry an anionic, a cationic, or an amphoteric charge, a feature which directly influences coating performance. Further, theresulting latex polymer can be blended with a range of other functional materials to impart additional or enhanced features to the final coating material.

In a number of applications, latex polymers can be blended with compositions containing bioactive compounds that exhibit antimicrobial activity, in order to provide a latex formulation that can be used in harsh environments where antimicrobial properties are particularly needed. These antimicrobial components are usually employed in relatively small amounts as formulating ingredients that are added after the polymer has been made. While such blends are useful, many practical issues remain in attempts to enhance or control the extent of antimicrobial protection these compositions might afford. For example, such compositions and methods are often inadequate for providing long-term protection of substrates or materials in which they are deployed, especially in their antifungal properties. Methods to augment or to more finely control the antimicrobial properties are also needed. Regulatory issues associated with introducing a new antimicrobial material, namely the polymer, may be significant. Moreover, approaches to prolong or extend the effectiveness of the antimicrobial properties remain elusive.

Therefore, what are needed are new methods and approaches to impart and to enhance antimicrobial activity of latex polymers, as well as the coatings and articles prepared therefrom. What are also needed are methods to more closely manage the antimicrobial activity of such materials, including approaches to extend the effectiveness of their bioactivity.

### SUMMARY OF THE INVENTION

This invention encompasses new methods and approaches to incorporate active ingredients, but not including bioactive components, such that the properties of the latex can be enhanced and controlled. As will be further discussed herein, the phrase "active ingredient" includes organic and inorganic components and should be construed in broad terms as an additive that provides a desired end benefit. As an example, an active ingredient of the present invention includes but is not limited to one or more moisturizing, anti aging, uv, tanning or antidandruff agents. However, the active ingredient does not include one or more bioactive components.

More explicitly, this invention also encompasses new methods and approaches to incorporate a variety of active ingredients. The present invention also relates to new types of active anionic polymer latex materials. In one aspect, this disclosure provides a method for incorporating active ingredients into a latex during the polymerization process.

Previously, antimicrobial agents have been added to a latex after the polymerization process and in relatively small amounts as preservatives for the latex product or for the end use application such as paints. The present invention allows the use of higher concentrations of a wide range of active ingredients, including highly hydrophobic active ingredients, which can be readily incorporated into the latices such that the resulting latex particles function as carriers for the active ingredients. The thorough incorporation of an active ingredient in this manner can afford a substantially homogeneous distribution of the additive and result in superior and sustained performance compared to pre-made dispersions.

In one aspect of this invention, an emulsion polymerization is carried out such that one or more active agents is incorporated into the polymer during the polymerization, typically by dissolving the respective one or more active components in a monomer stream. In this manner, the active agents can be at least partially encapsulated within the latex polymer matrix. The one or more active ingredients may be added to the monomer stream at any time during the polymerization process, however, those skilled in the art will recognize that certain active ingredients would benefit from addition late in the polymerization process to maintain the integrity and function of the active ingredient. One advantage provided by this process is the ability to incorporate or encapsulate large amounts of active ingredients, including hydrophobic components, without substantially degrading the respective active agent.

In another aspect, this invention also provides a tunable system based on an anionic latex which function as a type of carrier for at least one active ingredient, and optionally further including one or more active additives that can be blended with the latices disclosed herein. Thus, these latices can have a multifunctional purpose such as providing binding, strength, and dispersion properties in addition to being a carrier for an active functional ingredient.

In one aspect, because the active ingredients are typically incorporated into a latex during the emulsion polymerization process, these active components can be at least partially encapsulated within the latex polymer matrix. In another aspect, the active components can be substantially encapsulated within the latex polymer matrix. While not intending to be bound by one theory, it is believed that, by delivering the active ingredient to a desired end use application, the latex polymer with the encapsulated active ingredients can provide sustained and controlled exposure of the active ingredients to the environment in which they are deployed, thereby providing longer and more effective protection to the product or the application. Moreover, because both the active anionic latices described herein can be formed by existing emulsion polymerization processes, the polymerization methods advantageously allow for the preparation of high molecular weight polymers.

In yet a further aspect, the techniques disclosed herein can provide the ability to encapsulate larger amounts of the active ingredient into a latex composition than are afforded by standard methods.

While the methods disclosed herein can be applied to any active ingredient, including but not limited to either organic or inorganic agents, the present invention should be interpreted to encompass methods for providing or enhancing the properties of a latex, substrate, or particular end product through the encapsulation of any beneficial material, but not including bioactive components.

As used herein, the term "active" component includes, but is not limited to, UV agents, cosmeceuticals, cosmetics, oxides, minerals, and pigments. In other words, the term is used to include all ingredients capable of encapsulation that provide a benefit to the resulting latex composition. To the extent that any of the following active components are bioactive components, they are excluded from the present invention. As one example, a moisturizing agent is considered an active component or ingredient of the present invention. Similarly, a UV agent is considered an active component or ingredient of the present invention. Thus, the present invention further includes a latex that incorporates both a moisturizer and a UV agent.

In another aspect, this invention provides an active anionic polymer latex comprising:
a) a latex polymer comprising the polymerization product of: i) at least one ethylenically unsaturated first monomer; and ii) at least one ethylenically unsaturated second monomer that is anionic or a precursor to an anion;
b) at least one bioactive component at least partially encapsulated within the latex polymer; and
c) optionally, at least one sterically bulky component incorporated into the latex polymer.

While the inventive latices of this disclosure are anionic in nature, it is not necessary that the anionic charge of these latices be imparted by a monomer that is anionic or a precursor to an anion, that is, an anionic monomer. For example, an anionic initiator or an anionic surfactant that can be polymerizable or non-polymerizable can be used to introduce the anionic charge to the inventive latices.

When more than one ethylenically unsaturated first monomer is used to constitute the first monomer component, each of these first monomers is selected independently. Similarly, when more than one ethylenically unsaturated second monomer that is anionic or a precursor to a anion, referred to herein as the "anionic" monomer, is used to constitute the second monomer component, each of these second monomers is selected independently. In these aspects, a wide range of weight percentages of the at least one first monomer and the at least one second monomer can be used in this invention. For example, the latex can comprise from about 0.01 percent to 100 percent by weight of the ethylenically unsaturated first monomer, based on the total monomer weight, and the latex can comprise from 0 percent to about 99.99 percent by weight of the ethylenically unsaturated second monomer that is anionic or a precursor to an anion, based on the total monomer weight.

Further, the latices of this invention can also comprise a sterically bulky component which is incorporated into the anionic polymer latex to sterically stabilize the latex. These sterically bulky components can include, but are not limited to, monomers, polymers, and mixtures thereof as set forth below. Thus, a monomer can be incorporated as a comonomer that can attach to, or constitute a portion of, the backbone of the anionic polymer, examples of which include an alkoxylated ethylenically unsaturated third monomer. A polymer can be incorporated by adsorbing or being grafted onto the latex surface, an example of which includes polyvinyl alcohol.

Also, while the at least one sterically bulky component incorporated into the latex polymer is an optional component, this invention also provides for use of a wide range of amounts and concentrations of this component. Thus, as will be understood by the skilled artisan, in active anionic polymer latices that do not incorporate at least one sterically bulky component, latex stability can be enhanced by increasing the relative proportion of the anionic second monomer, by varying the amount and type of the initiator used, by the addition of surfactants such as nonionic or anionic surfactants, and the like, or any combination of such methods. The relative proportion of the anionic second monomer can be reduced and/or surfactants can be eliminated in the presence of at least one sterically bulky component.

In still another aspect, this invention provides a method of making an active anionic polymer latex comprising initiating an emulsion polymerization of an aqueous composition comprising, at any time during the emulsion polymerization:
a) at least one ethylenically unsaturated first monomer;
b) optionally, at least one ethylenically unsaturated second monomer that is anionic or a precursor to an anion;
c) at least one anionic surfactant;
d) at least one active component;
e) at least one free-radical initiator;
f) optionally, at least one sterically bulky ethylenically unsaturated third monomer;
g) optionally, at least one sterically bulky polymer; and
h) optionally, at least one nonionic surfactant, with the proviso that the active component is not a bioactive component.

In this aspect, because the anionic latices of this invention carry a net negative charge, when an anionic latex is prepared in the absence of the optional second anionic monomer, the overall negative charge of the latex can be imparted to the latex by a free radical initiator, by an anionic surfactant, by an anionic sterically bulky component, or by any combination thereof.

In one aspect of the invention, the at least one active component can be dissolved in the monomer feed at any time during the emulsion polymerization process. Further, in another aspect, the aqueous composition components and the at least one active component can be provided as a dispersion prior to initiating the emulsion polymerization. Thus, this invention provides for batch processes, in which the at least one active component is present in the seed stage. In this aspect, the emulsion polymerization is initiated when all the components of the composition, including the at least one active component, are present from the time of initiation. Further, this invention also provides for semi-continuous processes in which the emulsion polymerization is initiated at a time when all components of the composition are not present from the time of initiation, but some are added at various times after initiating the polymerization. In this aspect, for example, the at least one active component can be added at any time after the seed stage. In another aspect, for example, any other component or combination of components provided above can be added at any time after the seed stage, except for at least a portion of the total amount of any component that is required to initiate and propagate an emulsion polymerization. Thus, the active anionic latex provided herein can be made by any variety of batch or by a semi-continuous processes.

In one aspect, the active latices of this invention can be provided or used as a coating, which can be applicable to medical implants, including artificial ball and socket joints, rods, stents, dental implants, pins, screws, catheters, and the like. Such coatings can also be provided on everyday surfaces, such as air-conditioning coils, air filters, pipes, roofing, bathroom items, and kitchen items. Further examples of uses of the resultant products are use as an aqueous dispersion or directly in powder form, for example for indirect use, for example by addition to paints or other surface coatings.

Nonlimiting examples include odor control agents, moisturizing agents, anti-wrinkle and anti-aging agents, anti-dandruff agents, anti-static agents, preservatives, conditioners, styling aids, chelating agents, antioxidants, ultraviolet blockers and absorbers, skin bronzing or tanning agents, vitamins and herbal supplements, botanical extracts, free radical savengers, coloring agents, fragrances, and perfumes. Further, an active latex of the present invention may be used in the packaging of such applications.

These and other features, aspects, embodiments, and advantages of the present invention will become apparent after a review of the following detailed description of the invention. It should be understood, however, that these aspects, embodiments, and examples are provided for illustrative purposes only, and are not to be construed in any way as imposing limitations upon the scope thereof. Further, the present invention includes combinations of embodiments and aspects as herein provided.

### DETAILED DESCRIPTION OF THE INVENTION

The present invention provides new latex polymeric materials that can be used in a wide variety of end-uses, such as personal care products, including but not limited to skin and hair products, pharmaceuticals, cosmeceuticals, nutraceuticals, or as coatings on textiles, metal, cellulosic materials, and plastics, in which the polymeric materials include active components incorporated into the latex polymer. This invention also provides new methods and processes that allow incorporating high concentrations of an active ingredient during the emulsion polymerization. In one aspect, for example, the disclosed process can be used to incorporate from 0.01% to 40%, based on the total monomer weight ("phm" or parts per hundred of monomer), of a substantially hydrophobic ingredient during the emulsion polymerization.

Useful active additives can be solids, liquids, or combinations thereof. Many of the active additives that can be employed in this invention are substantially water insoluble or have limited solubility in water. In this aspect, the typical water insoluble, hydrophobic active agent can be soluble in at least one of the monomers employed in the emulsion polymerization. Thus, the typical hydrophobic active ingredient can be introduced into the polymerization reactor by substantially or partially dissolving it in a monomer feed at the appropriate time. In another aspect, useful active additives in this invention can also be substantially water soluble, one example of which includes o-phenylphenate (deprotonated o-phenylphenol), and similar agents. In this aspect, it is not necessary that such a hydrophilic active additive be soluble in any monomer that is to be polymerized.

In another aspect, it is not required that active ingredients be soluble in at least one of the monomers used, as these ingredients can also be added as a pre-made dispersion in water. In this aspect, the dispersions can be made, among other ways, by using a relatively concentrated amount of the additive and dispersing by using surfactants, dispersants, and the like, and typically employing a mixing device such as a high speed mixer, a homogenizer, an Eppenbach mixer, or similar devices. In such a case, the dispersion can be fed into the reactor to deliver the appropriate amount of active ingredient into the latex.

In one aspect, this invention encompasses an active anionic polymer latex comprising:
a) a latex polymer comprising the polymerization product of: i) at least one ethylenically unsaturated first monomer; ii) at least one ethylenically unsaturated second monomer that is anionic or a precursor to an anion;
b) at least one active component at least partially encapsulated within the latex polymer; and
c) optionally, at least one sterically bulky component incorporated into the latex polymer, with the proviso that the active component is not a bioactive component.

As provided herein, the at least one sterically bulky component incorporated into the latex polymer can be selected independently from at least one sterically bulky ethylenically unsaturated third monomer, at least one sterically bulky polymer, or any combination thereof. Each of these components, as well as optional or additional components, is considered herein.

In another aspect, this invention also encompasses a method of making an active anionic polymer latex comprising initiating an emulsion polymerization of an aqueous composition comprising, at any time during the emulsion polymerization:
a) at least one ethylenically unsaturated first monomer;
b) optionally, at least one ethylenically unsaturated second monomer that is anionic or a precursor to an anion;
c) at least one anionic surfactant;
d) at least one active component;
e) at least one free-radical initiator;
f) optionally, at least one sterically bulky ethylenically unsaturated third monomer;
g) optionally, at least one sterically bulky polymer; and
h) optionally, at least one nonionic surfactant, with the proviso that the active component is not a bioactive component.

In yet another aspect, this invention provides a method of making an active anionic polymer latex comprising:
a) providing an aqueous composition comprising:
   i) at least one ethylenically unsaturated first monomer;
   ii) optionally, at least one ethylenically unsaturated second monomer that is anionic or a precursor to an anion;
   iii) at least one anionic surfactant;
   iv) optionally, at least one sterically bulky ethylenically unsaturated third monomer;
   v) at least one free-radical initiator; and
   vi) optionally, at least one nonionic surfactant;
b) initiating an emulsion polymerization of the composition; and
c) adding at least one active component to the composition during the emulsion polymerization process, with the proviso that the active component is not a bioactive component.

In this aspect, at least one anionic surfactant is typically used to prepare the active anionic polymer latex. The at least one anionic surfactant that is employed can be in the form of an anionic surfactant that also does not constitute an ethylenically unsaturated second monomer, or the at least one anionic surfactant can be an ethylenically unsaturated second monomer that is anionic or a precursor to an anion. In the latter case, the second monomer that is anionic or a precursor to an anion functions both as an ethylenically unsaturated second monomer and as an anionic surfactant. In any event, when an anionic latex is prepared in the absence of the optional second anionic monomer, the overall negative charge of the latex can be imparted to the latex by a free radical initiator, by an anionic surfactant, by an anionic sterically bulky component, or by any combination thereof.

Many compounds and species that can be used as ethylenically unsaturated first monomers and sterically bulky components are disclosed in the European Patent Number EP 1109845 and the corresponding PCT Published Patent Application WO 00/8008077.

### Ethylenically Unsaturated First Monomers

Various ethylenically unsaturated first monomers can be used in the latex of the present invention. Examples of suitable first monomers can be found at least in U.S. Patent Number 5,830,934, U.S. Patent Application Publication Numbers 2005/0065284 and 200510003163, and European Patent Number EP 1109845, all to Krishnan. In this aspect, examples of such monomers include, vinyl aromatic monomers, halogenated or non-halogenated olefin monomers, aliphatic conjugated diene monomers, non-aromatic unsaturated mono- or dicarboxylic ester monomers, unsaturated alkoxylated monoester or diester monomers, unsaturated diesters of an acid anhydride monomer, nitrogen-containing monomers, nitrile-containing monomers, cyclic or acyclic amine-containing monomers, branched or unbranched alkyl vinyl ester monomers, aryl vinyl ester monomers, halogenated or non-halogenated alkyl (meth)acrylate monomers, halogenated or non-halogenated aryl (meth)acrylate monomers, carboxylic acid vinyl esters, acetic acid alkenyl esters, carboxylic acid alkenyl esters, a vinyl halide, a vinylidene halide, or any combination thereof, any of which having up to 20 carbon atoms. Thus, the ethylenically unsaturated first monomer is selected from a monomer that is not anionic and is not a precursor to an anion under the reaction and workup procedures.

In this aspect, it is the Applicant's intent to disclose both acrylate and methacrylate moieties when either moiety is disclosed in a suitable monomer. Thus, the disclosure that an acrylate monomer is a suitable ethylenically unsaturated first monomer also encompasses the disclosure that the corresponding methacrylate monomer is also a suitable first monomer. The abbreviation (meth)acrylate can be used to represent such a disclosure.

Many different ethylenically unsaturated first monomers can be used in preparing the active latices of this invention. In one aspect, suitable examples of ethylenically unsaturated first monomers include, styrene, para-methyl styrene, chloromethyl styrene, vinyl toluene, ethylene, butadiene, methyl (meth)acrylate, ethyl (meth)acrylate, propyl (meth)acrylate, butyl (meth)acrylate, pentyl (meth)acrylate, glycidyl (meth)acrylate, isodecyl (meth)acrylate, lauryl (meth)acrylate, (meth)acrylonitrile, (meth)acrylamide, N-methylol (meth)acrylamide, N-(isobutoxymethyl)(meth)acrylamide, vinyl neodecanoate, vinyl versatate, vinyl acetate, C3-C8 alkyl vinylethers, C3-C8 alkoxy vinyl ethers, vinyl chloride, vinylidene chloride, vinyl fluoride, vinylidene fluoride, trifluoroethylene, tetrafluoroethylene, chlorotrifluoroethylene, hexafluoropropylene, chlorotrifluoroethylene, perfluorobutyl ethylene, perfluorinated C3-C8 alpha-olefins, fluorinated C3-C8 alkyl vinylethers, perfluorinated C3-C8 alkyl vinylethers, and perfluorinated C3-C8 alkoxy vinyl ethers, or any combination thereof. Thus, halogenated analogs of suitable ethylenically unsaturated first monomers are encompassed by this disclosure, and it is Applicant's intent to disclose any and all suitable halogen-substituted analogs or derivatives of these monomers, including fluorine-substituted analogs, chlorine-substituted analogs, bromine-substituted analogs, and iodine-substituted analogs. The term "halogen-substituted" is meant to include partially halogen substituted and perhalogen substituted, in which any halogen substituents can be the same or can be different. In this aspect as well, it is the Applicant's intent to disclose both acrylate and methacrylate moieties when either moiety is disclosed in a suitable monomer.

In another aspect, the ethylenically unsaturated first monomer can be halogenated or can be non-halogenated. Similarly, the ethylenically unsaturated first monomer can be fluorinated or can be non-fluorinated. For example, fluorinated analogs of alkyl acrylates or methacrylates can be used, as well as the non-fluorinated compounds. The ethylenically unsaturated first monomer can also be chlorinated or can be non-chlorinated. The ethylenically unsaturated first monomer can also be brominated or can be non-brominated. The ethylenically unsaturated first monomer can also be iodinated or can be non-iodinated. For example, fluorinated analogs of alkyl acrylates or methacrylates can be used, as well as the non-fluorinated compounds.

In yet another aspect of this invention, the latices provided herein can comprise from 0.01 percent to 100 percent by weight of the ethylenically unsaturated first monomer, based on the total monomer weight. In this aspect, the latex of this invention can also comprise from 0.1 percent to 99.9 percent, from 1 percent to 99 percent, from 5 percent to 98 percent, from 10 percent to 95 percent, from 25 percent to 92 percent, from 35 percent to 90 percent, from 50 percent to 87 percent, or from 65 percent to 85 percent by weight of the ethylenically unsaturated first monomer, based on the total monomer weight. Suitable weight ranges of the at least one ethylenically unsaturated first monomer are a function of the design properties and the intended use of the material, as appreciated by the skilled artisan.

### Ethylenically Unsaturated Anionic Second Monomers

In still another aspect, the latex polymer of the present invention also comprises the polymerization product of at least one ethylenically unsaturated second monomer that is anionic or a precursor to an anion. As provided herein, the at least one ethylenically unsaturated second monomer can be collectively referred to by the term "anionic monomer," that is, any monomer which possesses or can be made to posses a negative charge. In one aspect, this negative charge may be imparted as a result of hydrolysis and formation of an acidic functionality that is readily deprotonated, or by way of another reaction known to one of ordinary skill that can result in a negatively-charged moiety. Such a reaction, for example a hydrolysis reaction, can take place at any stage in the emulsion polymerization process, such as in the component monomer, in an oligomer, in the resulting polymer, or any combination thereof. In another aspect, the negative charge may result from a pre-existing acid or salt functionality in the component monomer used to prepare the latex polymer. The anionic monomer is typically incorporated into the latex polymer by virtue of its ethylenic unsaturation.

Examples of suitable anionic monomers can be found at least in U.S. Patent Application Publication Numbers 2005/0065284 and 2005/0003163, to Krishnan. In this aspect, examples of suitable anionic monomers include, a monomer based on the half ester of an unsaturated dicarboxylic acid monomer, an unsaturated mono- or dicarboxylic acid monomer, a sulfate-containing monomer, a sulfonate-containing monomer, a phosphate-containing monomer, a phosphonate-containing monomer, an unsaturated anhydride, a monoester of an acid anhydride, or any combination thereof, any of which having up to 20 carbon atoms. When more than one ethylenically unsaturated second monomer is used to constitute the anionic monomer component, each anionic monomer is selected independently.

Further, suitable examples of ethylenically unsaturated anionic monomers that can be used in the latex of the present invention include, dimethylaminoethyl methacrylate, methoxypolyethyleneglycol methacrylate (meth)acrylic acid, maleic acid, maleic anhydride, 2-sulfoethyl (meth)acrylate, styrene sulfonate, 2-acrylamido-2-methylpropane sulfonic acid, monomethyl maleate, itaconic acid, itaconic anhydride, fumaric acid, or any combination thereof.

As described for the first monomers, halogenated analogs of suitable ethylenically unsaturated second monomers are also encompassed by this disclosure, and it is Applicant's intent to disclose any and all suitable halogen-substituted analogs or derivatives of these monomers, including fluorine-substituted analogs, chlorine-substituted analogs, bromine-substituted analogs, and iodine-substituted analogs. The term "halogen-substituted" is meant to include partially halogen substituted and perhalogen substituted, in which any halogen substituents can be the same or can be different. In this aspect as well, it is the Applicant's intent to disclose both acrylate and methacrylate moieties when either moiety is disclosed in a suitable monomer.

In a further aspect, the latex polymer of this invention can comprise from 0 to 99.99 percent by weight of the ethylenically unsaturated second monomer that is anionic or a precursor to an anion, based on the total monomer weight. In this aspect, the latex of this invention can also comprise from 0.01 to 99 percent, from 0.1 to 98 percent, from 0.5 to 95 percent, from 1 to 90 percent, from 2 to 80 percent, from 3 to 70 percent, from 4 to 60 percent, from 5 to 50 percent, from 7 to 40 percent, from 10 to 30 percent, or from 15 to 25 percent, by weight of the anionic second monomer, based on the total monomer weight.

### Sterically Bulky Components

As disclosed herein, one aspect of this invention encompasses an anionic polymer latex comprising: a) a latex polymer as disclosed herein; b) at least one active component at least partially encapsulated within the latex polymer; and c) optionally, at least one sterically bulky component incorporated into the latex polymer. In another aspect, this invention encompasses an anionic polymer latex comprising: a) a latex polymer as disclosed herein; b) at least one active component at least partially encapsulated within the latex polymer; and c) optionally, at least one sterically bulky component incorporated into the latex polymer. In yet another aspect, this invention encompasses an anionic polymer latex comprising: a) a latex polymer as disclosed herein; b) at least one bioactive component at least partially encapsulated within the latex polymer; and c) optionally, at least one sterically bulky component incorporated into the latex polymer.

The at least one sterically bulky component incorporated into the latex polymer can be selected independently from at least one sterically bulky ethylenically unsaturated third monomer, at least one sterically bulky polymer, or any combination thereof. In this aspect, and while not intending to be bound by theory, this sterically bulky component is typically incorporated into the anionic polymer latex to sterically stabilize the latex.

As used herein, the term "incorporated" with respect to the use of the at least one sterically bulky ethylenically unsaturated third monomer includes, the attachment of this third monomer to the anionic polymer, for example, by co-polymerization of the third monomer with the first monomer and the optional second monomer disclosed herein, to form the anionic polymer latex. Further, the term "incorporated" with respect to the at least one sterically bulky ethylenically unsaturated third monomer can also include the attachment of this third monomer to the anionic polymer in any other fashion, such as, for example, by grafting onto the polymer backbone. In another aspect, the term "incorporated" with respect to the use of the at least one sterically bulky polymer includes the attachment or association of this polymer into the latex for methods including, adsorbing or grafting the sterically bulky polymer onto the latex surface. For example, polyvinyl alcohol can be incorporated into the latex in this manner. This sterically stabilizing component can encompass a nonionic monomer or nonionic polymer which incorporate steric stabilization to the latex particle without affecting the deposition characteristics of the anionic polymer latex.

Exemplary monomers that can be used as sterically bulky ethylenically unsaturated third monomers include, those ethylenically unsaturated monomers that contain alkoxylated (for example, ethoxylated or propoxylated) functionalities. In one aspect, examples of such monomers include, but are not limited to, at least one a sterically bulky ethylenically unsaturated compound selected independently from the following:
a) CH2=C(R1A)COO(CH2CHR2AO)mR3A, wherein R1A, R2A, and R3A can be selected independently from H or an alkyl group having from 1 to 6 carbon atoms, inclusive, and m can be an integer from 1 to 30, inclusive. In this aspect, R1A, R2A, and R3A can also be selected independently from H or methyl, m can be an integer from 1 to 10, inclusive;
b) CH2=C(R1B)COO(CH2CH2O)n(CH2CHR2BO)pR3B, wherein R1B, R2B, and R3B can be selected independently from H or an alkyl group having from 1 to 6 carbon atoms, inclusive, and n and p can be integers selected independently from 1 to 15, inclusive. Also in this aspect, R1B, R2B, and R3B can be selected independently from H or methyl, and n and p can be integers selected independently from 1 to 10, inclusive;
c) CH2=C(R1C)COO(CH2CHR2CO)q(CH2CH2O)rR3C, wherein R1C, R2C, and R3C can be selected independently from H or an alkyl group having from 1 to 6 carbon atoms, inclusive, and q and r can be integers selected independently from 1 to 15, inclusive. Further to this aspect, R1C, R2C, and R3C can be selected independently from H or methyl, and q and r can be integers selected independently from 1 to 10, inclusive; or
d) any combination of any of these compounds.

In another aspect of this invention, a number of other types of unsaturated compounds can be used as sterically bulky ethylenically unsaturated third monomers including, polymerizable surfactants. Thus, further examples of suitable sterically bulky ethylenically unsaturated third monomers include, alkoxylated monoesters of a dicarboxylic acid; alkoxylated diesters of a dicarboxylic acid; alkyl allyl sulfosuccinate salts; vinyl sulfonate salts; polyoxyethylene alkylphenyl ethers such as NOIGEN RN™; polyoxyethylene alkylphenyl ethers ammonium sulfate such as HITENOL BC™; or any combination thereof. In this aspect, for example, ethoxylated mono- and diesters of diacids such as maleic and itaconic acids can also be used to achieve the desired stabilizing effect. Acrylate, methacrylate, vinyl and allyl analogs of surfactants, referred to as polymerizable surfactants, can also be used in this manner. Examples of such polymerizable surfactants include, TREM LF-40™ sold by Cognis. In one aspect, these surfactants are typical in that they possess ethylenic unsaturation that allows the surfactants to be incorporated into the latex polymer itself, as well as possessing hydrophobic and hydrophilic functionality that varies. In another aspect, surfactants that are particularly applicable to the present invention include the nonionic surfactants, wherein the hydrophilic character is believed to be attributable to the presence of alkylene oxide groups. Examples of suitable nonionic surfactants include, but are not limited to moieties derived from, ethylene oxide, propylene oxide, butylene oxide. In such species, the degree of hydrophilicity can vary based on the selection of functionality.

The at least one sterically bulky component incorporated into the latex polymer can also constitute at least one polymer. Again, while not intending to be bound by theory, it is thought that such polymers provide steric stability to the resulting latex polymer. Such polymers are sometimes referred to in the art as protective colloids. Examples of sterically bulky polymers include, polyvinyl alcohols, polyvinyl pyrollidone, hydroxyethyl cellulose, including any combination or derivative of these materials. Moreover, mixtures or combinations of any of the aforementioned sterically bulky monomers and any of these sterically bulky polymers can also be used as the at least one sterically bulky component that is incorporated into the latex polymer. A number of other monomers and polymers that can be used in the present invention that can impart stability are provided in U.S. Patent Number 5,830,934 to Krishnan et al.

The optional at least one sterically bulky component can be present in an amount ranging from 0 to 25 percent by weight, based on the total weight of the monomers. In this aspect, the latex of this invention can also comprise from 0.1 to 20 percent, from 0.2 to 18 percent, from 0.5 to 15 percent, from 0.7 to 12 percent, or from 1 to 10 percent by weight of the sterically bulky component, based on the total monomer weight.

### Free Radical Initiators

In still a further aspect, the latex of the present invention can include a free radical initiator that can initiate the emulsion polymerization, the selection of which is known to one of ordinary skill in the art. Because the anionic latices of this invention carry a net anionic charge, when an anionic latex is prepared in the absence of the optional second anionic monomer, the overall negative charge of the latex can be supplied by the free radical initiator. Thus, in addition to an anionic monomer, the overall negative charge can be imparted to the latex by a free radical initiator, by an anionic surfactant, by an anionic sterically bulky component, or by any combination thereof. Thus, while any anionic or nonionic free radical polymerization initiator can be used, and even low levels of a cationic initiator can be tolerated, typical free radical initiators include, anionic initiators including, persulfates, peroxides, azo-based compounds, or any combination thereof, that are capable of imparting an anionic charge to the resulting latex. In this aspect, any free radical initiator which generates an anionic species upon decomposition and contributes to the anionic charge of the latex can also be utilized. Examples of such an initiator include, 4,4'-azobis(4-cyano pentanoic acid), which is sold commercially as WAKO V-501™ by Wako Chemicals of Richmond, Virginia.

### Active Agents and Their Incorporation

The anionic latex polymerization and encapsulation method disclosed herein can be utilized with a wide range of active agents, alone or in combination. Anionic latex polymers can also be blended with compositions containing active compounds. To the extent that any of the following active components are bioactive components, they are excluded from the present invention.

In another aspect, this invention also provides methods to prepare an anionic latex fortified with an active component. To the extent that any of the following active components are bioactive components, they are excluded from the present invention. In one embodiment, the fortified latex is deposited through a wet end process onto pulp fibers. Such a deposition typically involves flocculation of the anionic latex using a cationic ingredient, which results in coagulation of the polymer onto the fiber, and provides a slurry of all the components that exhibits varying degrees of heterogeneity. In this aspect, the typical initiators also include azo-based compounds and compositions.

As provided herein, a wide range of polymerization conditions can be used. In one aspect, the active component or additive is typically soluble in at least one of the monomers employed, or soluble in a monomer mixture or composition used. In another aspect, the active additive can be introduced at any stage during the polymerization process including very early during the seed formation stage, including initiating the emulsion polymerization when all the components of the composition, including the at least one active component, are present at the time of initiation. In another aspect, a additive can be added during a later stage of polymerization process. For example, the active ingredient can be introduced into the monomer feed when 30 percent of the monomer has been fed into the polymerization reactor.

While not intending to be bound by theory, it is believed that introducing the active component into the monomer feed relatively late in the polymerization process could help minimize degradation of the active agent arising from the polymerization conditions. For example, it is possible that the active agent could be degraded at the temperature under which polymerization is conducted, or could react with certain monomers or other components. Accordingly, to minimize any such degradation process, the active agent can be added at such a time in the process, for example, when the process is more than 50%, more than 60%, more than 70%, more than 80%, or more than 90% complete, thus minimizing the contact time between the active agent and other components under the polymerization conditions. Another approach to minimize degradation of the active agent is to employ active agents that comprise "latent" active moieties that can be activated by thermal, chemical, photochemical, or similar means, at a suitable time after the emulsion polymerization.

In another aspect of this invention, the active additive can be introduced at any stage during an emulsion polymerization process, including, for example at such a time during the process at which the resulting latex exhibits an activity that is not substantially diminished relative to a standard activity exhibited by the same latex prepared by adding the bioactive component when the emulsion polymerization is 50% complete. Thus, this standard activity is the activity of the same latex synthesized from the same active component and the same latex at substantially the same concentrations, prepared by adding the active component when the emulsion polymerization is 50% complete, as evaluated under similar conditions. The term "not substantially diminished" is used to refer to any difference in activity of the resulting active latex, relative to this standard bioactivity, that meets any one, or more than one, of the following criteria: 1) the measured activity of the resulting active latex is less than or equal to 15% lower than the measured activity of the standard; 2) the activity of the resulting active latex has a numerical activity rating based on an arbitrary activity scale that is less than or equal to 35% lower than the numerical activity rating of the standard; or 3) the empirically-based descriptive rating of the activity level of the resulting active latex is no more than two descriptive rating levels lower than the activity rating level of the standard.

In another aspect, it is not necessary to introduce the active component into the monomer feed relatively late in the polymerization process. For example, the additive agent can also be added when 0 percent, 10 percent, 20 percent, 30 percent, 40 percent, 50 percent, 60 percent, 70 percent, 80 percent, 90 percent, or 100 percent of the monomer has been fed into the polymerization reactor. In this aspect, the emulsion polymerization is initiated at a time when all components of the composition are not present from the time of initiation, but some are added at various times after initiating the polymerization, including, the at least one active component.

In another aspect, polymerization can be effected at a range of temperatures, typically selected between the lowest temperature that affords reasonable polymerization rates, and the highest temperature allowable that does not result in substantial degradation or decomposition of the antimicrobial active ingredient. In one aspect, the polymerization can be carried out at the lowest temperature possible such that polymerization proceeds. In this case, the polymerization temperature should be sufficiently low to not substantially degrade or decompose any active ingredient that is incorporated, yet high enough such that polymerization rates and times are adequate for useful production of the final latex polymer.

The active agent can also be fed as a pre-emulsion made by emulsifying a mixture of monomer, additive, surfactants, and water, using methods and materials known to one of ordinary skill in the art. For example, in this aspect, the dispersions can be made by using a relatively concentrated amount of the additive and dispersing by using surfactants, and dispersants, and typically employing a mixing device such as a high speed mixer, a homogenizer, or an Eppenbach mixer. Moreover, any other conceivable process or process known to one of ordinary skill that provides emulsion polymers in which the additive is a dispersion, an emulsion, or a suspension or substantially dissolved in the monomer mixture prior to polymerization, can be utilized.

To the extent that any of the following active components are bioactive components, they are excluded from the present invention.

Additional active components that can be used in the present invention are provided in U.S. Patent Application Publication Number 2005/0003163, to Krishnan. Another aspect of this invention provides that the at least one active component can be selected independently from copper, copper salts, silver, silver salts, zinc, zinc salts, silver oxide, zinc oxide, chlorhexidine, chlorhexidine gluconate, glutaral, halazone, hexachlorophene, nitrofurazone, nitromersol, povidone-iodine, thimerosol, C1- to C5-parabens, hypochlorite salts, clofucarban, clorophene, poloxamer-iodine, phenolics, mafenide acetate, aminacrine hydrochloride, quaternary ammonium salts, oxychlorosene, metabromsalan, merbromin, dibromsalan, glyceryl laurate, pyrithione salts, sodium pyrithione, zinc pyrithione, (dodecyl) (diethylenediamine) glycine, (dodecyl) (aminopropyl) glycine, phenol, m-cresol, n-cresol, p-cresol, o-phenyl-phenol, resorcinol, vinyl phenol, polymeric guanidines, polymyxins, bacitracin, circulin, octapeptins, lysozmye, lysostaphin, cellulytic enzymes, vancomycin, ristocetin, actinoidins, avoparcins, tyrocidin A, gramicidin S, polyoxin D, tunicamycin, neomycin, streptomycin, or any combination thereof.

The composition of the invention may also include at least one post-added additive. The post-added additive may be at least one active component introduced to the latex composition or final formulation. The post-added additive may be a dispersion. To the extent that any of the following active components are bioactive components, they are excluded from the present invention.

In one embodiment, the at least one post-added additive can be an inorganic component such as an inorganic pigment selected independently from pigments such as titanium oxide or zinc oxide; black pigments, such as iron oxide black; fancy or multi-colored pigments, such as ultramarine or iron oxide red; lustrous pigments, metal effect pigments, pearlescent pigments as well as fluorescene or phosphorescent pigments; metal oxides, metal hydroxides and metal oxide hydrates, mixed phase pigments, sulfur-containing silicates, metal sulfides, complex metallo-cyanides, metal sulfates, metal chromates, metal molybdates, yellow iron oxide, brown iron oxide, manganese violet, sodium aluminum sulfosilicate, chromium oxide hydrate, ferric ferrocyanide, and cochineal. The inorganic component can also be at least one inorganic solids such as seed, broken seed nut shells, beads, luffa particles, polyethylene balls, clay, calcium bentonite, kaolin, china clay, talc, perlite, mica, vermiculite, silicas, quartz powder, montmorillonite, calcium carbonate, a talc or a member of the mica family or a chemical equivalent thereof, or any combination thereof. Still further, the at least one post-added additive can be a nano-inorganic material such as nano clays, nano oxides, or nanotubes. Further, although implied, the present invention includes any combination thereof.

In a further aspect of the present invention, the at least one post-added additive can be a hydrophobic component selected independently from natural plant-based waxes, animal derived waxes, natural and synthetic mineral waxes and synthetic waxes such as paraffin, carnauba, ozocertie, montan wax, polyolefin waxes such as, for example polybutylene, beeswax, or lanolin, candelilla and carnauba wax; alcohols comprising a carbon chain length of greater than two, preferably greater than four carbons, especially fatty alcohols such as cetyl alcohol, stearyl alcohol, cetostearyl alcohol, behenyl alcohol, propylene glycols, myristyl alcohols, arachidyl alcohol, lignoceryl alcohol; esters of the aforementioned alcohols such as stearates and myristates; metal stearates such as calcium stearate, zinc stearate, magnesium stearate or barium stearate; carboxylic acids such as caprylic acid, pelargonic acid, capric acid, undecylic acid, lauric acid, palmitic acid, behenic acid, terephthalic acid, phthalic acid, isophthalic acid, naphthalene-2,6-dicarboxylic acid, cyclohexanedicarboxylic acid, cyclohexanediacetic acid, succinic acid, adipic acid, and sebacic acid, especially carboxylic acids having a chain length greater than three carbons; fatty acids such as stearic acid, oleic acid, undecylenic acid and linoleic acid; oils such as perfume oils, essential oils, vegetable oil, fish oil, paraffin oil and mineral oil; fatty amides including primary amides such as stearamide, oleamide, erucamide, secondary amides such as stearyl stearamide, stearyl erucamide, bis amides such as ethylene bis stearamide, ethylene bis oleamide, alkanolamides such as coco mono ethanolamide, coco diethanolamide, oleic diethanolamide, lauric diethanolamide and stearic diethanolamide, as well as other various fatty amides such as aprylamide, pelargonamide, capramide, lauramide, myristamide, palmitamide, stearamide, arachidamide, behenamide, stearyl stearamide, palmitoleamide, oleamide, erucamide, linoleamide, linolenamide, oleyl palmitamide, stearyl erucamide, erucyl erucamide, oleyl oleamide, erucyl stearamide, and ricinoleamide; fatty bisamides such as ethylenebisstearamide, ethylenebisoleamide and ethylenebis 12-hydroxystearamide or any combination thereof.

In another aspect of the present invention, the at least one active component can be a cosmeceutical ingredient. For example, the active component may be a moisturizing or anti-wrinkle/anti-aging agent ingredient such as glycerin, propylene glycol, polyethylene glycol, hyaluronic acid, chondroitin sulfate, elastin, collagen, polysaccharide, glycosaminoglycan, ascorbic acid, ascorbic acid derivatives, glucosamine ascorbate, arginine ascorbate, lysine or tyrosine ascorbate, gluthathione ascorbate, nicotinamide ascorbate, niacin ascorbate, allantoin ascorbate, creatine ascorbate, creatinine ascorbate, chondroitin ascorbate, chitosan ascorbate, DNA ascorbate, carnosine ascorbate, tocotrienol, rutin, quercetin, hesperedin, diosmin, mangiferin, mangostin, cyanidin, astaxanthin, lutein, lycopene, resveratrol, tetrahydrocurcumin, rosmarinic acid, hypericin, ellagic acid, chlorogenic acid, oleuropein, alpha-lipoic acid, niacinamide lipoate, gluthathione, andrographolide, carnosine, niacinamide, polyphenols, pycnogenol and mixtures thereof; UV blocker and absorber ingredients such as benzophenones, benzotriazoles, salicylates, dibenzoylmethanes, anthranilates, methylbenzylidenes, octyl triazones, 2-phenylbenzimidazole-5-sulfonic acid, octocrylene, triazines, cinnamates, cyanoacrylates, dicyano ethylenes, etocrilene, drometrizole trisiloxane, bisethylhexyloxyphenol methoxyphenol triazine, drometrizole, dioctyl butamido triazone, terephthalylidene dicamphor sulfonic acid and para-aminobenzoates, salicylic acid, zinc pyrithione, as well as ester derivatives thereof; skin bronzing or tanning agent ingredients such as dihydroxyacetone, erytrulose, melanin; antioxidants such as vitamin C and derivatives thereof (e.g. ascorbyl acetate, ascorbyl phosphate and ascorbyl palmitate), vitamin A and derivatives thereof; folic acid and derivatives thereof; vitamin E and derivatives thereof such as tocopheryl acetate, flavons, or flavonoids, amino acids such as histidine, glycine, tyrosine, tryptophan and derivatives thereof; carotenoids and carotenes; uric acid and derivatives thereof; citric acid, lactic acid, malic acid; stilbenes and derivatives thereof; and pomegranate extracts; vitamin K1 or K2, vitamin K1 oxide or vitamin K2 oxide, hormones, minerals, plant or botanical extracts, anti-inflammatory agents, concentrates of plant extracts, emollients, skin protectants, humectants, silicones, skin soothing ingredients, analgesics or anti-itch agents, skin penetration enhancers, solubilizers, alkaloids and processing aids; coloring agents including various dyes and pigments; and perfumes or fragrances for the body or any combination thereof. In one embodiment of the active anionic polymer latex, a sunscreen may be formulated comprising at least one ultraviolet blocker synergistically used in combination with zinc oxide or titanium oxide to provide broader UV/Visible spectrum protection. The at least one ultraviolet blocker can be bound to the polymer or dispersed or encapsulated within the polymer.

The at least one active component can be a free radical scavenger such as cuprous halide, cupric halide, cupric acetate, cupric formate, cuprous acetate, cuprous formate, ferrous halide, ferric halide, ferrous sulfate, ferric sulfate, cysteine, glutathione, N-acetylcysteine, L-alpha-acetamido-beta mercaptopropionic acid, S-nitroso-glutathione, N-acetyl-3-mercapto-alanine, butylated hydroxyanisole, butylated hydroxytoluene, L-2-oxothiazolidine-4-carboxylate, desferrioxamine, allopurinol, superoxide dismutase and salen-manganese complexes and any combination thereof.

In yet another aspect of this invention, amounts of active component that can be added during the emulsion polymerization can range from 0.01 percent to 40 percent by weight active additive, based on the total monomer weight. In another aspect, amounts of active component that can be added during the emulsion polymerization can range from 0.025 percent to 35 percent, from 0.05 percent to 30 percent, from 0.1 percent to 25 percent, from 0.25 percent to 20 percent, or from 0.5 percent to 15 percent by weight active additive, based on the total monomer weight. In this aspect, the Applicant's intent is to disclose individually each possible number that such ranges could reasonably encompass, as well as any sub-ranges and combinations of sub-ranges encompassed therein. As compared to the amount of active component added as a "post-add," these concentrations of active additive are typically much larger than the post-add amounts. Among other things, this feature provides stable, concentrated dispersions that can be used as concentrates, as additives, or as concentrated dispersions that can be diluted and added to other systems which require the desired functionality, for example, moisturizing, or UV protection.

As disclosed herein, in one aspect, the active component is typically dissolved in the monomer feed during the emulsion polymerization process. Thus, the active additive is typically at least partially soluble in one or more of the monomers employed. Further, the active additive can be moderately soluble, substantially soluble, or highly soluble in one or more of the monomers employed. This feature can allow, among other things, the incorporation of hydrophobic active ingredients, the use of high amounts and concentrations of active ingredients, greater control over the properties of the active agent, the use of minimal amounts of surfactant, and at least partial encapsulation of the active ingredient. In some instances, the latex polymer can substantially encapsulate the added active component, thus the latex polymer can function as a type of carrier for the active ingredients. This process also allows for the incorporation of the active ingredients without substantially degrading the activity of these compounds.

In another aspect, useful active additives in this invention can also be water soluble to any extent, including substantially water soluble, examples of which include o-phenylphenate (deprotonated o-phenylphenol), glycerin, propylene glycol, and similar agents. Thus, it is not necessary that such a hydrophilic active additive be soluble in any monomer that is to be polymerized. In still another aspect, useful active additives in this invention can be substantially insoluble in the monomers being polymerized and substantially insoluble in water. In this aspect, a dispersion of the active component can be made by, dispersing a certain concentration of the additive with the use of surfactants, typically with the use of high speed mixers or homogenizers.

Because the post-added additives are typically dispersions that are post-mixed into a formulation, it can be difficult to adequately disperse the active additive into the polymer film, binder, coating, or the like, in which they are used. Moreover, typical additive dispersions that are used today can cause or be associated with moisture sensitivity and leaching of the additive, and many post-adds do not persist within the product for a sufficient period of time to provide adequate antifungal protection. The approach provided in this disclosure allows the use of minimal surfactants to incorporate the active additives into the latex, and because the additives are introduced during the polymerization, they are typically encapsulated and are not easily released from the resulting latex. As a result, there can be less leaching of the active component, and better overall distribution of the active ingredient throughout the polymer film, binder, coating, and the like. Accordingly, this method can provide a potentially safer and more environmentally friendly dispersion, while also offering sustained functionality. The active agent may also be released in a modified or controlled manner, if that is so desired, by appropriate selection of the polymer carrier and the active additive.

The process disclosed herein also allows the latex to be used as a concentrate, in contrast to the typical concentrate dispersions that are not as stable as those provided herein. As a result, the typical concentrate dispersions are not as easily manipulated and therefore cannot be incorporated as easily into a finished product, and can have deleterious effects on performance, such as water sensitivity, if dosage is increased. A concentrate of the latex provided herein can be diluted and used with or without other materials if such materials are needed to provide an adequate level of additive. Intimate incorporation of an active ingredient in this manner can afford a homogeneous distribution of the additive and result in superior and sustained performance compared to a pre-made dispersions.

An additional benefit of this intimate incorporation of the active agent is apparent in films that are prepared using these latices, which are observed to be substantially transparent. This feature highlights the highly homogeneous assimilation of the active agent into the latex particles and how this uniform distribution can provide useful properties for applications such as transparent active films, even in relatively high concentrations such as up to 20 percent to 25 percent. In one embodiment, the active ingredient can be released from the formulation, such as film, over a period of time (namely, modified or controlled release) and the period of release may depend on the surrounding conditions such as the pH of the environment where the polymer latex composition is utilized or the properties of the particular active ingredient. The particle size of the resulting polymer latex may be from 15 nm to 5 microns. More preferably, the particle size is from 20 nm to 2 microns and, most preferably, from 50 nm to 1 micron.

### Other Additives

In another aspect of this disclosure, the latex provided herein can also include other additives to improve the physical and/or mechanical properties of the polymer, the selection of which are known to one skilled in the art. Such additives include, for example, processing aids and performance aids, cross-linking agents, natural or synthetic binders, plasticizers, softeners, foam-inhibiting agents, froth aids, flame retardants, dispersing agents, pH-adjusting components, sequestering or chelating agents, or other functional components, or any suitable combination thereof.

### Polymer A

As will be appreciated by those skilled in the art, any anionic polymer latex may be used in the present invention. As one example, Polymer A represents a anionic polymer latex of the present invention.

| **Component Number** | **Batch Component** | **Charge Weight** |
|---|---|---|
| 1 | DW | 371.25 |
| 2 | Abex^{®} 2525 | 6.25 |
| 3 | DW | 281.25 |
| 4 | MPEG550MA | 10.00 |
| 5 | BA | 295.00 |
| 6 | STY | 185.00 |
| 7 | AA | 10.00 |
| 8 | DW | 5.00 |
| 9 | AP | 0.50 |
| 10 | DW | 50.00 |
| 11 | AP | 2.50 |
| 12 | DW | 4.72 |
| 13 | tBHP | 1.43 |
| 14 | DW | 4.73 |
| 15 | SFS | 1.00 |
| Total | | 1228.63 |

To prepare Polymer A, components 1 and 2 were charged to a reactor. Components 3 and 4 were charged to an aqueous monomer tank. Components 5, 6 and 7 were charged to the monomer tank. The initial and feed catalyst were prepared. Approximately 10% of each monomer was charge fed to reaction. The reaction vessel was purged w/ N₂ and heated to approximately 70°F. While holding at temp, components 8 and 9 were charged. The reaction was held for 30 min. The feeds were initiated, with aqueous monomer over approximately 5 hours, monomer over approximately 5 hours, and anionic over approximately 5.5 hrs. (330 min.) Components 12, 13, 14, and 15 were charged and the reaction was held for 15 min. The reaction is stripped, cooled and the solids were adjusted to 41-42%.

### Exemplary Substrates and Applications for Active Anionic Polymer Latices

The deposition of the latex polymer coatings of this disclosure on any number of different substrates, such as textiles, metal, cellulosic materials, and plastics, can impart desired end-use performance properties to those materials, and therefore tailor the substrates for a range of applications. For example, in one aspect, the present disclosure provides a treated fibrous material which can comprise at least one fiber and at least one active anionic polymer latex as provided herein. In one aspect, the treated fibrous material can comprise at least one fiber and at least one active anionic polymer latex deposited on, or associated with, the at least one fiber. If desired, the active anionic polymer can be applied to the fiber in the form of a powder, or the polymer composition can be deposited on the fiber by any suitable method known to the skilled artisan.

As used herein, the term "fiber" is intended to be broadly construed and can include single or multiple filaments that can be present in a variety of ways. It should be appreciated that only a single fiber can be treated with the active anionic polymer latex of the invention if so desired. Fibers that can be used in conjunction with this invention can encompass natural fibers, synthetic fibers, or any combination or mixture thereof. Natural fibers include, animal fibers (for example, silk and wool); mineral fibers (for example, asbestos); and vegetable-based fibers (for example, cotton, flax, jute, and ramie). Synthetic fibers include, but are not limited to, those made from polymers such as polyamides, polyesters, acrylics, and polyolefins. Other examples of fibers include, but are not limited to, rayon and inorganic substances extruded in fibrous form such as glass, boron, boron carbide, boron nitride, carbon, graphite, aluminum silicate, fused silica, and metals such as steel. In another aspect, cellulosic or wood fibers also can be treated with the active anionic polymer latex of the invention if so desired. Recycled fibers using any suitable fiber such as the above materials may also be employed. Any mixture of fibers can be treated with the active anionic polymer latex of the invention if so desired.

The treated fibrous material can, in another aspect, have at least one other polymeric layer deposited on the fiber so as to form a composite fibrous structure, thus multiple polymeric layers of various types can be used if desired. For example, anionic polymer latices may be deposited on the treated fibrous material to enhance specific properties of the treated fibrous material. While not intending to be bound by theory, it is thought that simple coulombic interactions between anionic and cationic polymers enhance the stability of such structures, leading to treated fibrous materials that are robust. Layers of various other polymers that do not contain any active agent can be employed similarly, for example, deposited on the anionic polymer latex which is present on the fibrous material to form a composite structure. In this fashion, unique layering architecture can be constructed with specially modified surfaces in accordance with this invention.

For the purposes of this disclosure, the term "substrate" is intended to be construed and interpreted broadly to include all those formed from inorganic materials, organic materials, composites thereof, mixtures thereof, or any type combination thereof. For example, the substrate can encompass, paper, composites, fibers, fillers, and pigments, as well as other organic and inorganic materials.

In one aspect of this invention, as disclosed herein, a fibrous substrate can be employed. The term "fibrous substrate" is also intended to be construed and interpreted broadly to include at least all the fibers, woven textiles, and non-woven textiles disclosed herein. Thus, the fibrous substrate may be present, for example, in the form of a web, a yarn, a fabric, a textile substrate. Further examples of fibrous substrates include, natural fibers such as cotton and wool to synthetic fibers such as nylon, acrylics, polyesters, and urethanes. Known application processes can be used to apply the bioactive anionic polymer latex, such as rod/knife coating, impregnation, back coatings, printing, as pretreatments on individual fibers, or as a finished good. Also as used herein, the term "textile substrate" can be defined according to its use in U.S. Patent Number 5,403,640 to Krishnan et al.. In this aspect, for example, "textile substrate" can encompass a fiber, a web, a yarn, a thread, a sliver, a woven fabric, a knitted fabric, a non-woven fabric, an upholstery fabric, a tufted carpet, and a pile carpet including any combination thereof, formed from any of the fibers described herein.

The active anionic latex composition of this invention also can be applied to a wide variety of plastic or rubber substrates. Examples of such materials include, commodity molded thermoplastics such as polyolefins; engineering thermoplastics such as polysulfones, acetals, polycarbonates; thermosets such as epoxies, urethanes, and related materials; and as extruded or blown films. The polymer could be applied as a coating on the surface by rod/knife coating, spray, dipping, as a laminate coating during the extrusion process, or as a coating applied in the mold during the molding process. Rubber products would include sheets, extruded/molded articles, and composites. In another aspect, the active anionic latex compositions of this invention also can be deployed in solid form. In this aspect, for example, the inventive latices can be coagulated or spray dried to provide the solid active anionic latex, which can be employed in solid form as an additive in plastic products, in processes such as extrusion or blow molding, as additives for various polyethylenes, and polypropylenes, and in any number of other polymer and plastic applications.

The active anionic latex composition of this invention also can be applied to wood or metal substrates. In this aspect, suitable substrates would include all kinds of natural and engineered wood substrates. Suitable metal substrates would indude both metals and metal alloys, such as carbon steel, stainless steel, and including solid steel bars, sheets, coils, ropes, and such, wherein the composition is applied as a coating by one of the numerous processes such as spraying dipping, brushing, roller coating, and related methods.

In this context, an article of manufacture comprising a substrate and an active anionic polymer latex deposited or positioned thereon can be made in accordance with standard procedures known to one of ordinary skill in the relevant art. The article of manufacture can have, in another aspect, at least one other polymeric layer deposited thereon so as to form a composite structure, thus multiple polymeric layers of various types can be used if desired. For example, other layers of various polymers can be deposited on the bioactive anionic polymer latex which is present in the article of manufacture to form a composite structure. Thus, uniquely tailored articles with specially modified surfaces can be made in accordance with the present invention.

In a broader aspect, the present invention also provides a coated material comprising any material and an active anionic polymer latex deposited or positioned thereon, wherein additional layers of other materials optionally can be used in combination with the active anionic polymer latex of this invention. As used herein, the term "material" is intended to be used broadly to include, any inorganic material, any organic material, any composite thereof, or any combination thereof. Examples of suitable materials include, a fiber, a filler, a particle, a pigment, composites thereof, combinations thereof, and mixtures thereof.

A multiple deposition process can also be used to make composite films that have applications in areas other than textiles and fibrous materials. Cellulosic structures can also be made using the anionic polymer latex provided herein including, cellulosic composites and heavy duty cellulosic structures. Examples of cellulosic composites include, those composites relating to filtration, shoe insoles, flooring felt, and gasketing. Heavy duty cellulosic structures include, dunnage bags, industrial wipes, and related structures. In a further aspect, the deposition process and anionic polymer latex of this invention also can be used in other technology arts including, anionic flocculants, wet and dry strength additives for papermaking, anionic retention aids, cement modifications, dye fixation, and redispersible powders.

The present invention can afford certain advantages as compared to previous methods used to fabricate active materials. In one aspect, for example, the active anionic latices can be substantially deposited on a substrate such that residual active latex does not remain in the processing fluid medium, providing a potential advantage from an environmental standpoint. Moreover, active anionic latices can be preferentially deposited on any substrate that carries a net positive charge, and deposition can occur in a uniform manner, thereby using less latex. In this aspect, and while not intending to be bound by theory, the active anionic latices are thought to be capable of forming substantially uniform monolayers of polymer material on a positively charged substrate, thereby allowing the use of less latex to provide the desired coverage. Because the active anionic latices can be formed by existing emulsion polymerization processes, the polymerization methods advantageously allow for the preparation of high molecular weight polymers.

Many fillers such as mica or calcium carbonate are negatively charged and can be difficult to use in large amounts in combination with cationic latices. Thus, when a filled latex is desired, this invention affords, among other things, an anionic latex polymer that can be used to prepare a filled latex, even when relatively high concentrations of fillers are needed.

As provided herein, the latex composition of the present invention can be applied to a wide variety of substrates using various techniques that are well known to one of ordinary skill in the art. As a result, there are numerous applications for the present invention, many of which are provided in the following listing. In this aspect, while this listing is not comprehensive, specific applications include: textiles such as residential and commercial carpets or tiles; liquid and air filters for HVAC or vacuum cleaners, or automotive uses; medical surgical gowns, drapes, dressings, and covers; pretreatment for fibers, printed or dyed fabrics for apparel, furnishings, sheets, and towels; diapers and incontinence articles; interior automotive applications such as trim, upholstery, mats, filters, and such; upholstery coatings; laminating and bonding adhesives; foams for sound absorbency; foamed articles such as pillows and mattresses; belting or other machinery parts for food handling; tapes such as masking tapes, surgical tape, industrial tapes electrical, industrial, and household cleaning wipes, cloths, and sponges; shoe products such as insoles, box toes, and such; plastic and/or rubber items such as tool handles, tool grips, toys, rubber gloves, sheets, or other articles; machinery housing such as for computers, display and diagnostic devices or instrumentation; medical devices such as catheters, balloons, tubing, syringes, and diagnostic kits, packaging or product protection, as applied to perishables, computer peripherals, semiconductors, memory chips, CDs, and DVDs; impact modifiers for acrylics, polycarbonates, and such; overdips or underdips for gloves such as gloves for clean rooms; breathable films; film former for fabric supported gloves; cutting boards; extruded and blown films for packaging; paper products such as vacuum bags, book covers, air filters, liquid filters, wallcoverings, wet and dry wipes, tissues, and such; felt for vinyl floor coverings; molded pulp applications; packaging such as boxes, cartons, molded articles, and related items; size press coatings for gift wraps, ink jet media, and breathable coatings; wet end additives in paper, tapes, labels for use in masking, surgical applications, general purpose applications, and such; binders for use in paper; binders for use in wallboard such as gypsum wallboard; adhesives for use in tapes, labels, decals, films, book bindings, pressure sensitive applications, flexible packaging and laminating adhesive (FPLA); inorganic and/or organic materials such as coating or encapsulation of fillers or pigments, construction sealers and grouts, gypsum wallboard coatings or binders, exterior or interior coatings; tile adhesives; floor coatings for use in hospitals, clean rooms, clinics, schools, and related environments; coatings for hospital and medical environments; ceiling tiles; glass fiber coatings such as glass mats, insulation, filter materials, reinforced composites, and such; coatings for air conditioning or refrigeration coils; other components for air conditioning systems, heat exchangers, ion exchangers, process water systems including cooling water treatment, solar-powered units, coated pipes; kitchen items; components of sanitary equipment; components of water systems; operator units of devices such as touch panels; materials used in bathrooms such as shower curtains, fixtures, toilet items, and even jointing or sealing compounds; medical devices such as use in coatings for stents, implants, prostheses, catheters, tubing, contact lenses, protective or backing films, medical instruments, and other medical devices for providing the sustained action of bioactive agents; articles which are contacted by large numbers of people such as telephone handsets, stair rails, door handles, window catches, grab straps and grab handles in public conveyances; liquid disinfectants and cleaners; personal care or hygiene products such as shampoos, lotions, creams, hair and skin care products, body wash, cosmetics, toilet items, and the like; hygiene coatings of surfaces other than floors, such as in hospitals, clinics, schools, homes, offices; hard and porous surface coatings as applicable to walls, ceilings, floors, counter tops; decorative concrete; wood such as oriented strand board (OSB) coatings; decking and construction materials for coating or impregnation; composite construction materials; furniture coatings; hygiene coatings such as used in table tops, counter tops, door knobs, door handles, fixtures, and the like; flooring applications such as in laminates, hardwood flooring, and other composite flooring materials; decorative laminates such as table tops, counter tops, furniture; other other construction materials such as roofing material, wall material, facades, fencing, or for wood protection applications; marine applications such as in boat hulls, docks, buoys, drilling platforms, or ballast water tanks; metal such as cabinets, door knobs, handles, fixtures, and such; and furniture, coatings as applicable to appliances, original equipment manufacture (OEM).

In addition, the latex polymer coatings containing at least one active component can be deposited on any number of different substrates to impart desired end-use performance properties to any of the aforementioned materials or provide a wide range of cosmeceutical or nutraceutical benefits. For example, in one aspect, the present polymer latex comprising at least one active component can be utilized in or as part of various moisturizing agents, anti-wrinkle agents and anti-aging agents, ultraviolet blockers and absorbers, skin bronzing or tanning agents, vitamins and herbal supplements, botanical extracts, free radical savengers, coloring agents, hair dyes, fragrances and perfumes.

### Applications of Active Anionic Polymer Latices in Wallboard Manufacture

Wallboard is typically produced by enclosing a core of an aqueous slurry prepared using calcium sulfate hemihydrate, referred to as calcined gypsum, and other materials between two large sheets of wallboard cover paper. After the gypsum slurry has set and has been dried, the formed sheet is cut into standard sizes. Thus, the core of wallboard can be considered to be prepared by combining a "dry" portion and a "wet" or aqueous portion which is then situated between two sheets of cover paper, and which sets or hardens.

A major "dry" ingredient of the gypsum wallboard core is calcium sulfate hemihydrate, commonly referred to as calcined gypsum or stucco, which is prepared by drying, pulverizing, and calcining natural gypsum rock (calcium sulfate dihydrate). The drying step simply removes any free moisture that is not chemically bound in the rock, while calcining liberates a portion of the chemically bound water molecules. As a result, calcined gypsum has the desirable property of being chemically reactive with water, and will set rather quickly when the two are contacted and the calcium sulfate hemihydrate is rehydrated to its dihydrate state. In addition to calcium sulfate hemihydrate, the dry ingredients can include a wide range of addititives, such as set retarders, set accelerators, antidesiccants, stabilizers, starch, or other additives, alone or in combination, that can be useful in the production process or the final wallboard properties.

In addition to including water, the "wet" portion of the wallboard core composition comprises paper pulp. In one aspect, the wet portion of the wallboard core composition typically, though not necessarily, includes a first wet component and a second wet component. The first wet component can be referred to as a paper pulp solution, and includes a mixture of water, paper pulp, optionally one or more fluidity-increasing agents, and optionally a set retarder. The paper pulp solution provides a major portion of the water that forms the gypsum slurry of the core composition. The second wet component can include a composition comprising strengthening agents, foaming agents, surfactants, other conventional additives, or any combination thereof. Any wet component generally, or the first wet component and second wet component, can be combined with the dry portion of the gypsum wallboard core in any order or manner.

In another aspect, the face paper and backing paper cover sheets used in wallboard manufacture are typically multi-ply paper manufactured from re-pulped newspapers Both the face paper and the backing paper usually have an inner ply (typically unsized) which contacts the core slurry such that gypsum crystals can grow up to or into the inner ply. This gypsum crystal-paper interaction constitutes one principal form of bonding between the core slurry and the cover sheet. The middle plies are usually sized and an outer ply is more heavily sized and can be treated to control the absorption of paints and scalers. Both cover sheets typically have sufficient permeability to allow for water vapor to pass through during the downstream board drying process. These and related methods for the production of gypsum wallboard generally are described, for example, in Michelsen, T. "Building Materials (Survey)," Kirk-Othmer Encyclopedia of Chemical Technology, (1992 4th ed.), vol. 4, pp. 618-619.

One aspect of this invention provides an active wallboard article of manufacture comprising at least one active latex polymer disclosed herein, and also provides a process for making an bioactive gypsum wallboard comprising at least one active latex polymer. In this aspect, the active latex polymer can be used in any component of the wallboard, that is, as a component of the gypsum wallboard core, the first cover sheet, the second cover sheet, or any combination thereof. Thus, this method and article comprise adding at least one active latex to the wallboard or any component thereof, at levels sufficiently effective against microbes, therefore, an active latex is an optional ingredient of each wallboard component. Moreover, the at least one active latex polymer can be used in any form, such as an emulsion, a dispersion, or in solid form, as disclosed herein. Thus in a further aspect, this disclosure provides for adding the at least one active latex polymer in a finishing step such as coating, spraying, or painting.

In a further aspect, this invention also provides for using active anionic polymer latices as binder or coating materials that can be combined with paper pulp used to prepare the face paper and backing paper cover sheets in wallboard manufacture. In one aspect, either or both sheets of the wallboard cover paper can comprise at least one active anionic polymer latex disclosed herein, which can be the same or can be different. These active anionic latices can be used to prepare the inner, middle, or outer plies of the cover sheets, or any combination thereof. Moreover, any combination of cover sheets in which the first, the second, or both covers sheets comprise active components such as antimicrobial components can be used with a gypsum slurry that comprises at least one active anionic polymer latex, or with a gypsum slurry that does not comprise at least one active anionic polymer latex.

Thus in one aspect, this disclosure provides a method of making a wallboard comprising:
a) forming a slurry comprising calcium sulfate hemihydrate, water, paper pulp, and optionally at least one first active anionic polymer latex;
b) depositing the slurry onto a first cover sheet optionally comprising at least one second active anionic polymer latex; and
c) applying a second cover sheet optionalty comprising at least one third active anionic polymer latex on top of the deposited slurry; and
d) drying the resulting wallboard;
wherein at least one of the slurry, the first cover sheet, or the second cover sheet comprises at least one active anionic polymer latex; and
wherein the at least one first active anionic polymer latex, the at least one second active anionic polymer latex, and the at least one third active anionic polymer latex are the same or are different.

Thus, the at least one first, the at least one second, and at least one third active anionic polymer latices are selected independenty of each other. Any of the active anionic polymer latices or combinations of active anionic polymer latices disclosed herein can be employed in any of the wallboard components.

Accordingly, this invention also provides a wallboard comprising:
a) a gypsum sheet optionally comprising at least one first active anionic polymer latex;
b) a first cover sheet disposed on one side of the gypsum sheet and optionally comprising at least one second active anionic polymer latex; and
c) a second cover sheet disposed on the opposite side of the gypsum sheet and optionally comprising at least one third active anionic polymer latex;
wherein at least one of the gypsum sheet, the first cover sheet, or the second cover sheet comprise at least one active anionic polymer latex; and
wherein the at least one first active anionic polymer latex, the at least one second active anionic polymer latex, and the at least one third active anionic polymer latex are the same or are different.

The at least one first, the at least one second, and at least one third active anionic polymer latices are selected independently of each other. The wallboard compnents can comprise any of the active anionic polymer latices or combinations of active anionic polymer latices disclosed herein.

The present invention is further illustrated by the following examples, which are not to be construed in any way as imposing limitations upon the scope thereof. On the contrary, it is to be clearly understood that resort can be had to various other aspects, embodiments, modifications, and equivalents thereof which, after reading the description herein, may suggest themselves to one of ordinary skill in the art without departing from scope of the appended claims.

In the following examples, unless otherwise specified, the reagents were obtained from commercial sources. Reference to reagents may include reference to a generic description, a brand or trade name, or both. General procedures, including general synthetic testing procedures for polymer latices, are provided in U.S. Patent Application Publication Numbers 2005/0065284 and 2005/0003163, to Krishnan.

### DEMONSTRATIVE EXAMPLE 1

As one of ordinary skill in the art appreciates, deodorant compositions may comprise a variety of chemical components in various amounts. Table 1 sets forth a demonstrative deodorant composition and the amounts of each component This demonstrative deodorant composition may be prepared by first combining components 1 and 3. Next, the preparer may slowly add the resulting mixture into component 2 in the presence of agitation and heat (75°C) and then add component 4 to the resulting batch and mix the batch until component 4 dissolves. Next, the preparer slowly adds component 5 to the batch, mixes the batch until component 5 dissolves, and then cools the batch to a temperature of 45°C. The preparer then adds components 6-7 to the batch and mixes until a uniform batch results. Lastly, the preparer homogenizes the batch at 4500 rpm for 10 minutes resulting in an deodorant formulation. Such deodorant compositions may be formulated as a roll-on, stick or spray and may, optionally, be combined with an antiperspirant.

**Table 1**

| **Component No.** | **Component** | **% Weight** | **Batch Size** |
|---|---|---|---|
| 1 | DC 245 Fluid (Dow Corning) (cyclopentasiloxane) | 49.30 | 493.00 |
| 2 | Bentone Gel® VS-5/PC (propylene carbonate) | 13.50 | 135.00 |
| 3 | Puresyn 4TM (hydrogenated C6-14 olefin polymers) | 10.00 | 100.00 |
| 4 | AsensaTM CL 110 (polyethylene) | 1.00 | 10.00 |
| 5 | Cabosil® M5 (silica) | 0.20 | 2.00 |
| 6 | ReachTM AZP 908 SUF (aluminum zirconium chlorhydrate) | 24.00 | 240.00 |
| 7 | Dipropylene Glycol | 2.00 | 20.00 |
| Total | | 100.00 | 1000.00 |

### DEMONSTRATIVE EXAMPLE 2

Body wash formulations may comprise a variety of chemical components in various amounts. Table 2 sets forth a demonstrative body wash formulation and the amounts of each component. This demonstrative body wash formulation may be prepared by dissolving component 2 in component 1. Next, the preparer adds component 3, mixes and heats (75°C) the resulting batch to form a first phase. The preparer then combines components 4 and 5, heats to 70°C and mixes until the batch fully melts to form a second phase. Next, the preparer adds the second phase into the first phase with agitation and mixes until a uniform batch results. The preparer may then add components 6-8 one by one into the batch with mild agitation and cool to 40°C. Next, the preparer adds components 9 to the batch, mixes the batch and adjusts the pH to 6.0-6.5 with component 10, as needed. Finally, the preparer adjusts the viscosity to 7,000-15,000 CPS with a 20% NaCl solution, as needed. Within 30 minutes of preparation, the viscosity of the formulation of the present example was determined using a Brookfield RVT#4 at 20 RPM, 30 sec. At 12 hours post-preparation, viscosity was again determined using a Brookfield RVT#5 at 20 RPM, 30 sec.

**Table 2**

| **Component No.** | **Component** | **% Weight** | **Batch Size** |
|---|---|---|---|
| 1 | Deionized Water | 49.21 | 492.08 |
| 2 | Na₂EDTA | 0.10 | 1.00 |
| 3 | Butylene Glycol | 2.00 | 20.00 |
| 4 | Monamid^{®} CMA (cocamide MEA) | 2.00 | 20.00 |
| 5 | Stepan^{®} EGMS (glycol stearate) | 1.50 | 15.00 |
| 6 | Standapol^{®} A (ammonium lauryl sulfate) | 25.00 | 250.00 |
| 7 | Standapol^{®} ES-2 (sodium laureth sulfate) | 15.00 | 150.00 |
| 8 | Velvetex^{®} BK-35 (cocamidopropyl betaine) | 5.00 | 50.00 |
| 9 | Shampoo Fragrance#3599 | 0.15 | 1.50 |
| 10 | Citric Acid | 0.04 | 0.42 |
| Total | | 100.00 | 1000.00 |

### DEMONSTRATIVE EXAMPLE 3

As one of ordinary skill in the art appreciates, shampoo formulations may comprise a variety of chemical components in various amounts. Table 3 sets forth a demonstrative shampoo formulation (control) and the amounts of each component. This demonstrative shampoo formulation may be prepared by first combining components 1-5 (first phase) and heating the resulting phase to a temperature of 75°C with slow mixing. Next, the preparer may combine components 6-7 (second phase) and heat the resulting phase to a temperature of 75°C with slow mixing. The preparer then adds the second phase to the first phase and mixes the two phases until a uniform batch at room temperature results. Next, components 8-9 may be added to the batch one at a time. Finally, the pH of the resulting batch may be adjusted to 6.0-6.5 with component 10.

**Table 3**

| **Component No.** | **Component** | **% Weight** | **Batch Size** |
|---|---|---|---|
| 1 | Water | 36.69 | 366.88 |
| 2 | Na₂EDTA | 0.05 | 0.50 |
| 3 | Bioterge AS 40 (sodium C₁₄₋₁₆ Olefin Sulfonate) | 45.00 | 450.00 |
| 4 | Glucamate DOE 120 (PEG-120 Methyl Glucose Dioleate) | 1.50 | 15.00 |
| 5 | Zemea^{®} Propanediol | 2.00 | 20.00 |
| 6 | Monamid^{®} CMA (cocamide MEA) | 3.00 | 30.00 |
| 7 | Velvetex ^{®} BK-35 (cocamidopropyl betaine) | 10.00 | 100.00 |
| 8 | Kathon^{®} CG (methylisothiazolinone) | 0.06 | 0.60 |
| 9 | Mackpearl^{®} DR-140V (cocamide MEA) | 1.50 | 15.00 |
| 10 | Citric Acid | 0.20 | 2.02 |
| Total | | 100.00 | 1000.00 |

### SYNTHETIC EXAMPLE 4

### Active Anionic Latex Preparation

A one-liter polymerization reactor can be charged with the following ingredients: about 270 g of water; about 6 g of the nonionic surfactant Abex^{®} 2525 (Rhodia); about 2.7 g of an anionic surfactant Dowfax™ 2A1 (Dow Chemical Company), and about 3 g of methacrylic acid. The reactor contents can be deoxygenated by subjecting the reactor to several vacuum / N2 fill cycles.

The following reactor feeds can be prepared:
1) An aqueous monomer feed containing about 150 g of water, about 6 g of methoxy polyethyleneglycolmethacrylate (MPEG 550 from Cognis), about 4.5 g of methacrylic acid, about 1.3 g of Dowfax™ 2A1, and about 6 g of Abex^{®} 2525. The total feed time into the reactor is 5 hours;
2) A non-aqueous monomer feed containing about 153 g of butyl acrylate, about 132 g of methyl methacrylate, and about 64 g of the bioactive agent. The total feed time for this feed is 5 hours. The bioactive agent can be introduced into this feed after about a 3-hour time period. Thus, the non-aqueous monomer feed during the first 3 hours contains only butyl acrylate and methyl methacrylate; and
3) An initiator feed that can contain about 30 g of water and about 2.10 g of an initiator, V-501™ (Wako Chemical). The total feed time is about 5.5 hours. A few drops of ammonia can be added to aid in the dissolution of the initiator, if needed.

To the initial reactor charge can be added 10% of the non-aqueous monomer feed, which contains only the two monomers methyl methacrylate and butyl acrylate, as the bioactive agent is not introduced into the monomer until 3 hours into the feed. The temperature of the reactor then can be raised to about 165 °F and when this set point is reached, an original initiator solution (separate from the initiatorfeed described above) containing about 3 g of water and about 0.30 g of V-501 can be injected into the reactor. The reactor contents are maintained at this temperature for about 30 minutes before the feeds are started.

When addition of the feeds is completed, the reaction is continued until most (greater than about 98%) of the monomers have reacted. The reactor contents then can be cooled down and the vacuum stripped to remove unreacted monomers and to raise the solids concentration to about 42-43 percent by weight. If necessary, the pH of the latex can be adjusted to around 6.0 to about 7.0 before stripping the reaction volatiles.

### Reference SYNTHETIC EXAMPLE 5

### Active Anionic Latex Prepared by Late Introduction of a Bioactive Agent

An emulsion polymerization reaction can be conducted according to the details provided in Example 2, except that an approximately 32 g-sample of bioactive component can be introduced into the non-aqueous monomer feed after about 4 hours, rather than 3 hours, of the 5-hour non-aqueous monomer feed.

Experiments were conducted to demonstrate the incorporation of various active ingredients. The active ingredients are incorporated into the polymer during the emulsion polymerization process by dissolving the active components in the monomer stream. One of ordinary skill in the art will appreciate that one or more latex polymers may be utilized in the resulting composition.

### SYNTHETIC EXAMPLE 6

Experiments were conducted to evaluate the incorporation of zinc oxide (Nyacol® DP370) into a polymer latex formulation. Table 4 sets forth an anionic latex formulation and the amounts of each component The resulting latex formulation can be used as a sunscreen. In the present example, components 1-4 were charged to the reaction vessel. An aqueous monomer feed (components 6-10) and monomer feed (components 11-12) premix were prepared. An initial catalyst (component 15) and feed catalyst (component 16) were prepared. The pH was then adjusted to 6.5 with approximately 2 ml of NH4OH. The reaction was then purged with inert gas (nitrogen) and heated to a temperature of 71°C. Once the temperature was attained, the initial catalyst was added and the reaction was held for 30 minutes. Next, the monomer (components 11-12) was fed for 5 hours. The aqueous monomer (components 6-10) was then fed into the reaction for 4 hours followed by the delayed catalyst feed (component 16) for 5.5 hours. After 4 hours, the pH was adjusted to 6.7 with approximately 2 ml of NH3, the temperature was raised to 75°C and a one hour feed of components 13-14 was initiated. Lastly, the pH of the resulting formulation was adjusted to 7.0 with NH3, 300 ml of DW were added and stripped to 53% TS. The resulting physical properties are summarized in Table 5.

The components listed in the tables below are abbreviated using ordinary conventions. Definitions for some terms are provided. If a particular abbreviation is not specifically defined herein, the abbreviation should not be considered indefinite but rather used within the ordinary vernacular of those skilled in the art.

| | |
|---|---|
| DW -- deionized water; | AWC -- sodium formaldehyde sulfoxylate; |
| Abex^{®} 2525 -- nonionic surfactant; | MAA -- methacrylic acid; |
| MPEG550MA -- methoxypolyethyleneglycol methacrylate; | Dowfax™ 2A1 -- surfactant; |
| | IA -- itaconic acid; |
| Special NMA -- proprietary blend of N- methylol acrylamide and acrylamide; | SFS -- sodium formaldehyde sulfoxylate; |
| | NaP -- sodium persulfate; |
| BA -- butyl acrylate; | STY -- styrene; |
| MMA -- methyl methacrylate; | ST -- styrene; |
| Wako V501 -- 4,4'-azo bis-4- cyanopentanoic acid; | Dissolvine NA3-36 -- ethylenediaminetetraacetic acid, trisodium salt |
| TBHP -- tertiarybutylhdroperoxide; | |

**Table 4**

| Component No. | Component | Charge Weight |
|---|---|---|
| 1 | DW | 350.00 |
| 2 | Abex^{®} 2525 | 12.50 |
| 3 | MAA | 5.00 |
| 4 | Dowfax™ 2A1 | 5.56 |
| 5 | Dissolvine NA36 | 6.94 |
| 6 | DW | 50.00 |
| 7 | MPEG550MA | 10.00 |
| 8 | Dowfax™ 2A1 | 2.22 |
| 9 | Abex^{®} 2525 | 25.00 |
| 10 | MAA | 7.50 |
| 11 | BA | 257.50 |
| 12 | MMA | 220.00 |
| 13 | DW | 40.00 |
| 14 | Nyacol^{®} DP370 | 20.83 |
| 15 | WakoV501 | 2.50 |
| 16 | WakoV501 | 40.00 |
| Total | | 1055.56 |

**Table 5**

| Final Physical Properties | |
|---|---|
| Actual % Solids | 50.10 |
| % Conversion | 99.0 |
| Particle Size (nm) | 184 |
| Viscosity (CPS) | 46 |
| pH | 8.0 |

### SYNTHETIC EXAMPLE 7

Experiments were conducted to evaluate the incorporation of clay (montmorillonite) into a polymer latex formulation. Table 6 sets forth an anionic latex formulation and the amounts of each component. The resulting latex formulation can be used in various cosmetics as a thickener or potentially as a crack or wrinkles filler in cosmetic applications. In the present example, a batch was mixed at high speed for at least 20 minutes to allow the clay to be completely dispersed. Components 1-5 were charged in a reactor and heated to 95°C. The monomer feed (components 10-13) was initiated and fed at a rate of 12.5% for the first thirty minutes and then the remaining 87.5% was fed from 30 minutes until 195 minutes. Lastly, components 6-9 were fed into the reactor over 220 minutes. The reactor contents are then adjusted to a pH of 5.5-6.5 with a 15% NaOH solution and stripped to remove unreacted monomers and raise solids. The resulting physical properties of the stripped formulation are summarized in Table 7.

**Table 6**

| Component No. | Component | Charge Weight |
|---|---|---|
| 1 | DW | 487.86 |
| 2 | IA | 8.30 |
| 3 | seed latex | 3.01 |
| 4 | Dissolvine NA3-36 | 0.28 |
| 5 | Montmorillonite | 10.00 |
| 6 | DW | 139.05 |
| 7 | NaP | 5.50 |
| 8 | Dowfax^{™} 2A1 | 5.56 |
| 9 | Sodium Hydroxide | 1.60 |
| 10 | STY | 275.000 |
| 11 | BA | 199.368 |
| 12 | Sulfole 120 | 0.50 |
| 13 | DW | 4.79 |
| 14 | TBHP | 0.71 |
| 15 | DW | 5.00 |
| 16 | SFS | 0.50 |
| Total | | 1147.03 |

**Table 7**

| Final Physical Properties | |
|---|---|
| Actual % Solids | 46.9 |
| Particle Size (nm) | 215 |
| Viscosity (CPS) | 1088 |
| pH | 3.18 |

### SYNTHETIC EXAMPLE 8

Another experiment were conducted to evaluate the incorporation of clay (montmorillonite) into a polymer latex formulation. Table 8 sets forth an anionic latex formulation and the varying amounts of each component. In the present example, components 1-3 were combined and mixed 30 minutes to form a premix. The premix was added to a reactor and mixed at 200 RPM. The monomer comprising components 4-6 was then added to the reactor (10%) and the reactor was purged for 15 minutes. Next, the reaction was heated to 70°C and the initial catalyst (components 7-8) was added to the reactor. The reaction was held for 30 minutes. Next, the monomer (components 4-6) was fed for 4 hours. The aqueous monomer (components 9-10) was then fed into the reaction for 3 hours followed by the delayed catalyst feed (components 11-12) for 4.5 hours. Next, the temperature was increased to 75°C after 1.5 hours. After the feeds concluded, the reaction was held for 30 minutes. Next, a first treatment (components 13-16) was fed to the reaction over 30 minutes and held for 10 minutes. A second treatment (components 17-20) was fed to the reaction over 30 minutes and the reaction was sampled for any residual monomer. Next, the reaction was held again for 10 minutes and then cooled to room temperature. The resulting physical properties are summarized in Table 9.

**Table 8**

| Component No. | Component | Charge Weight |
|---|---|---|
| 1 | DW | 600.00 |
| 2 | Dowfax 2A1 | 1.67 |
| 3 | Montmorillonite | 15.00 |
| 4 | BA | 120.00 |
| 5 | ST | 177.00 |
| 6 | MAA | 3.00 |
| 7 | DW | 3.00 |
| 8 | AP | 0.60 |
| 9 | DW | 60.00 |
| 10 | Dowfax^{™} 2A1 | 13.33 |
| 11 | DW | 60.00 |
| 12 | AP | 1.50 |
| 13 | DW | 3.00 |
| 14 | TBHP | 0.86 |
| 15 | DW | 3.00 |
| 16 | SFS | 0.60 |
| 17 | DW | 3.00 |
| 18 | TBHP | 0.86 |
| 19 | DW | 3.00 |
| 20 | SFS | 0.60 |
| Total | | 1070.01 |

**Table 9**

| Final Physical Properties | |
|---|---|
| Actual % Solids | 30.5 |
| % Conversion | 100.0 |
| Particle Size (nm) | 90 |
| Viscosity (CPS) | 470 |
| pH | 2.6 |

### SYNTHETIC EXAMPLE 9

Experiments were conducted to evaluate the incorporation of zinc pyrithione (3% level) (available as Zinc Omadine^{®} dispersion) into a polymer latex formulation. Table 10 sets forth an anionic latex formulation and the amounts of each component In the present example, components 1-4 were charged to the reaction vessel. An aqueous monomer feed (components 6-10) and monomer feed (components 11-12) premix were prepared. An initial catalyst (component 15) and feed catalyst (component 16) were prepared. The pH was then adjusted to 6.5 with approximately 2 ml of NH₄OH. The reaction was then purged with inert gas (nitrogen) and heated to a temperature of 71°C. Once the temperature was attained, the initial catalyst was added and the reaction was held for 30 minutes. Next, the monomer (components 11-12) was fed for 5 hours. The aqueous monomer (components 6-10) was then fed into the reaction for 4 hours followed by the delayed catalyst feed (component 16) for 5.5 hours. After 4 hours, the pH was adjusted to 6.7 with approximately 2 ml of NH₃, the temperature was raised to 80°C and a one hour feed of components 13-14 was initiated. Lastly, the pH of the resulting formulation was adjusted to 7.0 with NH₃, 300 ml of DW were added and stripped to 53% TS. The resulting physical properties are summarized in Table 11.

**Table 10**

| Component No. | Component | Charge Weight |
|---|---|---|
| 1 | DW | 350.00 |
| 2 | Abex^{®} 2525 | 12.50 |
| 3 | MAA | 5.00 |
| 4 | Dowfax^{™} 2A1 | 5.56 |
| 5 | Dissolvine NA36 | 6.94 |
| 6 | DW | 50.00 |
| 7 | MPEG550MA | 10.00 |
| 8 | Dowfax^{™} 2A1 | 2.22 |
| 9 | Abex 2525 | 25.00 |
| 10 | MAA | 7.50 |
| 11 | BA | 255.00 |
| 12 | MMA | 220.00 |
| 13 | DW | 40.00 |
| 14 | Zinc Omadine^{®} | 31.25 |
| 15 | WakoV501 | 2.50 |
| 16 | WakoV501 | 40.00 |
| Total | | 1063.47 |

**Table 11**

| Final Physical Properties | |
|---|---|
| Actual % Solids | 48.40 |
| % Conversion | 95.4 |
| Particle Size (nm) | 155 |
| Viscosity (CPS) | 56 |
| pH | 7.1 |

### PROPHETIC EXAMPLE 10

A deodorant composition comprising at least one anionic polymer component can be prepared according to the method of Demonstrative Example 1 comprising the components set forth in Table 12. The deodorant may contain about 2.5% of Polymer A which encapsulates an active component (40% active).

**Table 12**

| **Component No.** | **Compenent** | **% Weight** | **Batch Size** |
|---|---|---|---|
| 1 | DC 245 Fluid (Dow Corning) (cyclopentasiloxane) | 46.80 | 468.00 |
| 2 | Bentone Gel® VS-5/PC (propylene carbonate) | 13.50 | 135.00 |
| 3 | Puresyn 4TM (hydrogenated C6-14 olefin polymers) | 10.00 | 100.00 |
| 4 | Asensa TM CL 110 (polyethylene) | 1.00 | 10.00 |
| 5 | Cabosil® M5 (silica) | 0.20 | 2.00 |
| 6 | Reach TM AZP 908 SUF (aluminum zirconium chlorhydrate) | 24.00 | 240.00 |
| 7 | Dipropylene Glycol | 2.00 | 20.00 |
| 8 | Polymer A (40% Active) | 2.50 | 2.50 |
| Total | | 100.00 | 1000.00 |

### SYNTHETIC EXAMPLE 11

In the present example, a base body wash formulation was prepared according to the method of Demonstrative Example 2 comprising the components set forth in Table 13. The preservative, Glydant® (DMDM Hydantoin), was mixed with component 10 and added to the batch just before pH was measured. To determine foam height, 5 grams of product and 145 grams of water were weighed and added into a blender. The product and water was grated for 10 seconds and poured into a 1000 ml graduated cylinder. The foam level was read, followed by a 2 minutes waiting period, and then the liquid level was read. To determine foam density, 10 grams of product and 145 grams of water were weighed and added into a blender. The product and water was grated for 10 seconds and the resulting foam was poured into a 100 ml graduated cylinder. A rubber stopper was then dropped into the graduated cylinder at which time a timer was started when the stopper reached the 80 ml mark. The timer was stopped when the stopper reached the 30 ml mark. The time was then recorded. Foam drainage was determined based on the amount of liquid collected at the bottom of the graduated cylinder once the stopper reached the 30 ml mark.

**Table 13**

| **Component No.** | **Component** | **% Weight** | **Batch Size** |
|---|---|---|---|
| 1 | Deionized Water | 49.01 | 490.08 |
| 2 | Na₂EDTA | 0.10 | 1.00 |
| 3 | Butylene Glycol | 2.00 | 20.00 |
| 4 | Monamid^{®} CMA (cocamide MEA) | 2.00 | 20.00 |
| 5 | Stepan^{®} EGMS (glycol stearate) | 1.50 | 15.00 |
| 6 | Standapol^{®} A (ammonium lauryl sulfate) | 25.00 | 250.00 |
| 7 | Standapol^{®} ES-2 (sodium laureth sulfate) | 15.00 | 150.00 |
| 8 | Velvetex^{®} BK-35 (cocamidopropyl betaine) | 5.00 | 50.00 |
| 9 | Glydant^{®} (DMDM hydantoin) | 0.20 | 2.00 |
| 10 | Shampoo Fragrance #3599 | 0.15 | 1.50 |
| 11 | Citric Acid | 0.04 | 0.42 |
| Total | | 100.00 | 1000.00 |

### SYNTHETIC EXAMPLE 12

In the present example, a base body wash formulation was prepared containing 0.2% polyquarternium-10, such as that sold under the tradename Polymer JR 400, without glycol stearate. The polyquarternium-10 was dispersed in water and mixed until hydrated before adding components 1-3 set forth in Table 14. The body wash was then prepared according to the method set forth in Demonstrative Example 2. The viscosity, foam height, foam drainage, and foam density were measured according to the methods set forth in Synthetic Example 11.

**Table 14**

| **Component No.** | **Component** | **% Weight** | **Batch Size** |
|---|---|---|---|
| 1 | Deionized Water | 48.63 | 486.32 |
| 2 | Na₂EDTA | 0.10 | 1.00 |
| 3 | Butylene Glycol | 2.00 | 20.00 |
| 4 | Polymer JR 400 (polyquaternium-10) | 0.20 | 2.00 |
| 5 | Monamid®CMA (cocamide MEA) | 2.00 | 20.00 |
| 6 | Standapol® A (ammonium lauryl sulfate) | 25.00 | 250.00 |
| 7 | Standapol® ES-2 (sodium laureth sulfate) | 15.00 | 150.00 |
| 8 | Velvetex® BK-35 (cocamidopropyl betaine) | 5.00 | 50.00 |
| 9 | Shampoo Fragrance #3599 | 0.15 | 1.50 |
| 10 | Citric Acid | 0.04 | 0.42 |
| 11 | NaCl (20% solution) | 1.92 | 19.18 |
| Total | | 100.00 | 1000.00 |

### SYNTHETIC EXAMPLE 13

In the present example, a base body wash formulation was prepared containing 0.2% polyquarternium-10 according the method set forth in Demonstrative Example 2. Table 15 sets forth the body wash formulation of the present example and the amounts of each component. The viscosity, foam height, foam drainage and foam density were measured according to the methods set forth in Synthetic Example 11.

**Table 15**

| **Component No.** | **Component** | **% Weight** | **Batch Size** |
|---|---|---|---|
| 1 | Deionized Water | 48.98 | 489.76 |
| 2 | Na₂EDTA | 0.10 | 1.00 |
| 3 | Butylene Glycol | 2.00 | 20.00 |
| 4 | Polymer JR 400 (polyquaternium-10) | 0.20 | 2.00 |
| 5 | Monamid^{®} CMA (cocamide MEA) | 2.00 | 20.00 |
| 6 | Stepan^{®} EGMS (glycol stearate) | 1.50 | 15.00 |
| 7 | Standapol^{®} A (ammonium lauryl sulfate) | 25.00 | 250.00 |
| 8 | Standapol^{®} ES-2 (sodium laureth sulfate) | 15.00 | 150.00 |
| 9 | Velvetex^{®} BK-35 (cocamidopropyl betaine) | 5.00 | 50.00 |
| 10 | Shampoo Fragrance #3599 | 0.15 | 1.50 |
| 11 | Citric Acid | 0.04 | 0.42 |
| 12 | NaCl (20% solution) | 0.03 | 0.32 |
| Total | | 100.00 | 1000.00 |

### PROPHETIC EXAMPLE 14

A body wash formulation may be prepared containing 2.5% of Polymer A (40% encapsulated active; no glycol stearate) according the method set forth in Demonstrative Example 2. Table 16 sets forth the body wash formulation of the present prophetic example and the amounts of each component The viscosity, foam height, foam drainage, and foam density may be measured according to the methods set forth in Synthetic Example 11.

**Table 16**

| **Component No.** | **Component** | **% Weight** | **Batch Size** |
|---|---|---|---|
| 1 | Deionized Water | 45.86 | 458.60 |
| 2 | Na₂EDTA | 0.10 | 1.00 |
| 3 | Butylene Glycol | 2.00 | 20.00 |
| 4 | Polymer A (40% Active) | 2.50 | 25.00 |
| 5 | Monamid^{®}CMA (cocamide MEA) | 2.00 | 20.00 |
| 6 | Standapol^{®} A (ammonium lauryl sulfate) | 25.00 | 250.00 |
| 7 | Standapol^{®} ES-2 (sodium laureth sulfate) | 15.00 | 150.00 |
| 8 | Velvetex^{®} BK-35 (cocamidopropyl betaine) | 5.00 | 50.00 |
| 9 | Shampoo Fragrance #3599 | 0.15 | 1.50 |
| 10 | Citric Acid | 0.04 | 0.42 |
| 11 | NaCl (20% solution) | 2.35 | 23.48 |
| Total | | 100.00 | 1000.00 |

### PROPHETIC EXAMPLE 15

Another base body wash formulation may be prepared containing a 2.5% Polymer A (40% encapsulated active) according the method set forth in Demonstrative Example 2. Table 17 sets forth the body wash formulation of the present prophetic example and the amounts of each component. The viscosity, foam height, foam drainage and foam density may be measured according to the methods set forth in Synthetic Example 11.

**Table 17**

| **Component No.** | **Component** | **% Weight** | **Batch Size** |
|---|---|---|---|
| 1 | Deionized Water | 44.88 | 448.78 |
| 2 | Na₂EDTA | 0.10 | 1.00 |
| 3 | Butylene Glycol | 2.00 | 20.00 |
| 4 | Polymer A (40% Active) | 2.50 | 25.00 |
| 5 | Monamid® CMA (cocamide MEA) | 2.00 | 20.00 |
| 6 | Stepan® EGMS (glycol stearate) | 1.50 | 15.00 |
| 7 | Standapol® A (ammonium lauryl sulfate) | 25.00 | 250.00 |
| 8 | Standapol® ES-2 (sodium laureth sulfate) | 15.00 | 150.00 |
| 9 | Velvetex® BK-35 (cocamidopropyl betaine) | 5.00 | 50.00 |
| 10 | Shampoo Fragrance #3599 | 0.15 | 1.50 |
| 11 | Citric Acid | 0.04 | 0.42 |
| 12 | NaCl (20% solution) | 1.83 | 18.30 |
| Total | | 100.00 | 1000.00 |

### PROPHETIC EXAMPLE 16

Shampoo formulations may be prepared comprising at least one polymer such as Polymer A (40% encapsulated active). In the present prophetic example, a shampoo formulation may be prepared according the method set forth in Demonstrative Example 3 and contain an anionic polymer. Table 18 sets forth the prophetic shampoo formulation and the amounts of each component. Viscosity can be determined using a Brookfield RVT#5 at 20 RPM. To determine foam height, 5 grams of product and 145 grams of water can be weighed and added into a blender. The product and water can be grated for 10 seconds and poured into a 1000 ml graduated cylinder. The foam level can be read, followed by a 2 minutes waiting period, and then the liquid level can be read. To determine foam density, 10 grams of product and 145 grams of water can be weighed and added into a blender. The product and water can be grated for 10 seconds and the resulting foam can be poured into a 100 ml graduated cylinder. A rubber stopper can then be dropped into the graduated cylinder at which time a timer can be started when the stopper reaches the 80 ml mark. The timer is then stopped when the stopper reaches the 30 ml mark. The time is then recorded. Foam drainage is determined based on the amount of liquid collected at the bottom of the graduated cylinder once the stopper reaches the 30 ml mark.

**Table 18**

| **Component No.** | **Component** | **% Weight** | **Batch Size** |
|---|---|---|---|
| 1 | Water | 34.12 | 341.23 |
| 2 | Na₂EDTA | 0.05 | 0.50 |
| 3 | Bioterge AS 40 (sodium C₁₄₋₁₆ Olefin Sulfonate) | 45.00 | 450.00 |
| 4 | Glucamate DOE 120 (PEG-120 Methyl Glucose Dioleate) | 1.50 | 15.00 |
| 5 | Zemea^{®} Propanediol | 2.00 | 20.00 |
| 6 | Polymer A (40% Active) | 2.50 | 25.00 |
| 7 | Monamid^{®} CMA (cocamide MEA) | 3.00 | 30.00 |
| 8 | Velvetex^{®} BK-35 (cocamidopropyl betaine) | 10.00 | 100.00 |
| 9 | Kathon^{®} CG (methylisothiazolinone) | 0.06 | 0.60 |
| 10 | Mackpearl^{®} DR-140V (cocamide MEA) | 1.50 | 15.00 |
| 11 | Citric Acid | 0.27 | 2.67 |
| Total | | 100.00 | 1000.00 |

### SYNTHETIC EXAMPLE 17

In the present example, another a shampoo formulation was prepared according to Demonstrative Example 3 and contained a fragrance but no antimicrobial polymeric material. Table 19 sets forth the shampoo formulation and the amounts of each component. The pH of the resulting batch was adjusted to 6.69 with component 10. The viscosity, foam height, foam drainage and foam density were measured according to the tests outlined in Prophetic Example 16.

**Table 19**

| **Component No.** | **Component** | **% Weight** | **Batch Size** |
|---|---|---|---|
| 1 | Water | 38.06 | 390.56 |
| 2 | Na₂EDTA | 0.05 | 0.50 |
| 3 | Bioterge AS 40 (sodium C₁₄₋₁₆ Olefin Sulfonate) | 45.00 | 450.00 |
| 4 | Glucamate DOE 120 (PEG-120 Methyl Glucose Dioleate) | 1.50 | 15.00 |
| 5 | Zemea^{®} Propanediol | 2.00 | 20.00 |
| 6 | Monamid^{®} CMA (cocamide MEA) | 1.50 | 15.00 |
| 7 | Velvetex ^{®} BK-35 (cocamidopropyl betaine) | 10.00 | 100.00 |
| 8 | Kathon CG (methylisothiazolinone) | 0.06 | 0.60 |
| 9 | Mackpearl^{®} DR-140V (cocamide MEA) | 1.50 | 15.00 |
| 10 | Citric Acid | 0.13 | 1.32 |
| 11 | Mardi Gras #5544 (fragrance) | 0.20 | 2.00 |
| Total | | 100.00 | 1000.00 |

### PROPHETIC EXAMPLE 18

Another shampoo formulation may be prepared according to the method set forth in Demonstrative Example 3 that contains a fragrance and Polymer A (encapsulated active). Table 20 sets forth a prophetic shampoo formulation and the amounts of each component The pH of the resulting batch can be adjusted to 6.66 with component 11. The viscosity, foam height, foam drainage and foam density can be measured according to the tests outlined in Prophetic Example 16.

**Table 20**

| **Component No.** | **Component** | **% Weiqht** | **Batch Size** |
|---|---|---|---|
| 1 | Water | 35.52 | 355.17 |
| 2 | Na₂EDTA | 0.05 | 0.50 |
| 3 | Bioterge AS 40 (sodium C₁₄₋₁₆ Olefin Sulfonate) | 45.00 | 450.00 |
| 4 | Glucamate DOE 120 (PEG-120 Methyl Glucose Dioleate) | 1.50 | 15.00 |
| 5 | Zemea^{®} Propanediol | 2.00 | 20.00 |
| 6 | Polymer A | 2.50 | 25.00 |
| 7 | Monamid^{®} CMA (cocamide MEA) | 1.50 | 15.00 |
| 8 | Velvetex ^{®} BK-35 (cocamidopropyl betaine) | 10.00 | 100.00 |
| 9 | Kathon CG (methylisothiazolinone) | 0.06 | 0.60 |
| 10 | Mackpearl^{®} DR-140V (cocamide MEA) | 1.50 | 15.00 |
| 11 | Citric Acid | 0.17 | 1.73 |
| 12 | Mardi Gras #5544 (fragrance) | 0.20 | 2.00 |
| Total | | 100.00 | 1000.00 |

### PROPHETIC EXAMPLE 19

Any variety of the active components disclosed herein may be encapsulated in the anionic latex polymers in any amount to achieve the desired result. For example, the following active components typically can be encapsulated from about 1 % to about 2% or more based on parts per hundred monomer (phm): organic UV filters such as benzophenones, benzotriazoles, homosalates, alkyl cinnamates, for example, octylmethoxycinnamate, octyl salicylate; self-tanning active components such as dihydroxyacetone (DHEA); moisturizing agents such as aloe vera extracts; and free radical scavengers such as vitamin A, C, and E, and other antioxidants such as phenolic antioxidants, for example, BHT (butylated hydroxytoluene) and BHA (butylated hydroxy anisole); carotenoids and carotenes; uric acid and derivatives thereof; citric acid, lactic acid, malic acid; stilbenes and derivatives thereof; and pomegranate extracts; vitamin K1 or K2, vitamin K1 oxide or vitamin K2 oxide, hormones, minerals, plant or botanical extracts, anti-inflammatory agents, concentrates of plant extracts, emollients, skin protectants, humectants, silicones, skin soothing ingredients, analgesics or anti-itch agents, skin penetration enhancers, solubilizers, alkaloids and processing aids; coloring agents including various dyes and pigments; and perfumes or fragrances for the body or any combination thereof.

## Claims

1. A method of making an active anionic polymer latex comprising initiating an emulsion polymerization of an aqueous composition comprising, at any time during the emulsion polymerization:
a) at least one ethylenically unsaturated first monomer;
b) optionally, at least one ethylenically unsaturated second monomer that is anionic or a precursor to an anion;
c) at least one anionic surfactant;
d) at least one active component;
e) at least one free-radical initiator;
f) optionally, at least one sterically bulky ethylenically unsaturated third monomer;
g) optionally, at least one sterically bulky polymer; and
h) optionally, at least one nonionic surfactant,
with the proviso that the at least one active component is not a bioactive component.

2. The method of making an active anionic polymer latex according to Claim 1, wherein the method is a semi-continuous process, and wherein at least one active component is dissolved in the monomer feed at any time during the emulsion polymerization.

3. The method of making an active anionic polymer latex according to Claim 1, wherein the method is a batch process, and wherein the at least one active component is present in the seed stage of the emulsion polymerization.

4. The method of making an active anionic polymer latex according to Claim 1, wherein the at least one ethylenically unsaturated first monomer is styrene, para-methyl styrene, chloromethyl styrene, vinyl toluene, ethylene, butadiene, methyl (meth)acrylate, ethyl (meth)acrylate, propyl (meth)acrylate, butyl (meth)acrylate, pentyl (meth)acrylate, glycidyl (meth)acrylate, isodecyl (meth)acrylate, lauryl (meth)acrylate, (meth)acrylonitrile, (meth)acrylamide, N-methylol (meth)acrylamide, N-(isobutoxymethyl)(meth)acrylamide, vinyl neodecanoate, vinyl versatate, vinyl acetate, a C3-C8 alkyl vinylether, a C3-C8 alkoxy vinyl ether, vinyl chloride, vinylidene chloride, vinyl fluoride, vinylidene fluoride, trifluoroethylene, tetrafluoroethylene, chlorotrifluoroethylene, hexafluoropropylene, chlorotrifluoroethylene, perfluorobutyl ethylene, a perfluorinated C3-C8 alpha-olefin, a fluorinated C3-C8 alkyl vinylether, a perfluorinated C3-C8 alkyl vinylether, a perfluorinated C3-C8 alkoxy vinyl ether, or a combination thereof.

5. The method of making an active anionic polymer latex according to Claim 1, wherein the at least one ethylenically unsaturated second monomer is (meth)acrylic acid, maleic acid, maleic anhydride, 2-sulfoethyl (meth)acrylate, styrene sulfonate, 2-acrylamido-2-methylpropane sulfonic acid, monomethyl maleate, itaconic acid, itaconic anhydride, fumaric acid, dimethylaminoethyl methacrylate, methoxypolyethyleneglycol methacrylate or a combination thereof.

6. A method of making an active anionic polymer latex according to claim 1, the method comprising:
a) providing an aqueous composition comprising:
i) at least one ethylenically unsaturated first monomer;
ii) optionally, at least one ethylenically unsaturated second monomer that is anionic or a precursor to an anion;
iii) at least one anionic surfactant;
iv) optionally, at least one sterically bulky ethylenically unsaturated third monomer;
v) at least one free-radical initiator; and
vi) optionally, at least one nonionic surfactant;
b) initiating an emulsion polymerization of the composition; and
c) adding at least one active component to the composition during the emulsion polymerization process,
with the proviso that the at least one active component is not a bioactive component.

7. An active anionic polymer latex comprising:
a) a latex polymer comprising the polymerisation product of:
i) at least one ethylenically unsaturated first monomer; and
ii) at least one ethylenically unsaturated second monomer that is anionic or a precursor to an anion;
b) at least one active component at least partially encapsulated within the latex polymer; and
c) optionally, at least one sterically bulky component incorporated within the latex polymer,
with the proviso that the at least one active component is not a bioactive component.

8. The active anionic polymer latex of claim 7, wherein the composition is capable of forming a film.

9. The active anionic polymer latex of claim 8, wherein the film controls the release of the at least one active component.

10. The active anionic polymer latex of claim 7, wherein the at least one ethylenically unsaturated first monomer is a combination of styrene and butyl acrylate.

11. The active anionic polymer latex of claim 7, wherein the at least one ethylenically unsaturated second monomer is dimethylaminoethyl methacrylate or methacrylic acid or methoxypolyethyleneglycol methacrylate.

12. The active anionic polymer latex of claim 7, wherein at least a portion of the at least one active component is post-added to the latex composition as a dispersion.

## Patentansprüche

1. Verfahren zur Herstellung eines aktiven anionischen Polymerlatex, das das Starten einer Emulsionspolymerisation einer wässrigen Zusammensetzung umfasst, die zu jeder Zeit während der Emulsionspolymerisation Folgendes umfasst:
a) mindestens ein ethylenisch ungesättigtes erstes Monomer;
b) optional mindestens ein ethylenisch ungesättigtes zweites Monomer, das anionisch oder ein Vorläufer eines Anions ist;
c) mindestens ein anionisches oberflächenaktives Mittel;
d) mindestens eine Wirkkomponente;
e) mindestens einen Radikalstarter;
f) optional mindestens ein sterisch voluminöses, ethylenisch ungesättigtes drittes Monomer;
g) optional mindestens ein sterisch voluminöses Polymer; und
h) optional mindestens ein nichtionisches oberflächenaktives Mittel;
mit der Maßgabe, dass die mindestens eine Wirkkomponente keine bioaktive Komponente ist.

2. Verfahren zur Herstellung eines aktiven anionischen Polymerlatex nach Anspruch 1, worin das Verfahren ein halbkontinuierlicher Prozess ist und worin mindestens eine Wirkkomponente in der Monomerzuführung zu jeder Zeit während der Emulsionspolymerisation gelöst ist.

3. Verfahren zur Herstellung eines aktiven anionischen Polymerlatex nach Anspruch 1, worin das Verfahren ein diskontinuierlicher Prozess ist und worin die mindestens eine Wirkkomponente in der Keimphase der Emulsionspolymerisation vorliegt.

4. Verfahren zur Herstellung eines aktiven anionischen Polymerlatex nach Anspruch 1, worin das mindestens eine ethylenisch ungesättigte erste Monomer Folgendes ist: Styrol, para-Methylstyrol, Chloromethylstyrol, Vinyltoluol, Ethylen, Butadien, Methyl(meth)acrylat, Ethyl(meth)acrylat, Propyl(meth)acrylat, Butyl(meth)acrylat, Pentyl(meth)acrylat, Glycidyl(meth)acrylat, Isodecyl(meth)acrylat, Lauryl(meth)acrylat, (Meth)acrylnitril, (Meth)acrylamid, N-Methylol(meth)acrylamid, N-(Isobutoxymethyl)(meth)acrylamid, Vinylneodecanoat, Vinylversatat, Vinylacetat, ein C3-C8-Alkylvinylether, ein C3-C8-Alkoxyvinylether, Vinylchlorid, Vinylidenchlorid, Vinylfluorid, Vinylidenfluorid, Trifluorethylen, Tetrafluorethylen, Chlortrifluorethylen, Hexafluorpropylen, Chlortrifluorethylen, Perfluorbutylethylen, ein perfluoriertes C3-C8-alpha-Olefin, ein fluorierter C3-C8-Alkylvinylether, ein perfluorierter C3-C8-Alkylvinylether, ein perfluorierter C3-C8-Alkoxyvinylether oder eine Kombination davon.

5. Verfahren zur Herstellung eines aktiven anionischen Polymerlatex nach Anspruch 1, worin das mindestens eine ethylenisch ungesättigte zweite Monomer Folgendes ist: (Meth)acrylsäure, Maleinsäure, Maleinsäureanhydrid, 2-Sulfoethyl(meth)acrylat, Styrolsulfonat, 2-Acrylamido-2-methylpropansulfonsäure, Monomethylmaleat, Itaconsäure, Itaconsäureanhydrid, Fumarsäure, Dimethylaminoethylmethacrylat, Methoxypolyethylenglycolmethacrylat oder eine Kombination davon.

6. Verfahren zur Herstellung eines aktiven anionischen Polymerlatex nach Anspruch 1, wobei das Verfahren Folgendes umfasst:
a) Bereitstellen einer wässrigen Zusammensetzung, die Folgendes umfasst:
i) mindestens ein ethylenisch ungesättigtes erstes Monomer;
ii) optional mindestens ein ethylenisch ungesättigtes zweites Monomer, das anionisch oder ein Vorläufer eines Anions ist;
iii) mindestens ein anionisches oberflächenaktives Mittel;
iv) optional mindestens ein sterisch voluminöses, ethylenisch ungesättigtes drittes Monomer;
v) mindestens einen Radikalstarter; und
vi) optional mindestens ein nichtionisches oberflächenaktives Mittel;
b) Starten einer Emulsionspolymerisation der Zusammensetzung; und
c) Zugeben von mindestens einer Wirkkomponente zur Zusammensetzung während des Emulsionspolymerisationsprozesses,
mit der Maßgabe, dass die mindestens eine Wirkkomponente keine bioaktive Komponente ist.

7. Aktiver anionischer Polymerlatex, der Folgendes umfasst:
a) ein Latexpolymer, das das Polymerisationsprodukt von Folgenden umfasst:
i) mindestens einem ethylenisch ungesättigten ersten Monomer; und
ii) mindestens einem ethylenisch ungesättigten zweiten Monomer, das anionisch oder ein Vorläufer eines Anions ist;
b) mindestens eine Wirkkomponente, die zumindest teilweise innerhalb des Latexpolymers eingekapselt ist; und
c) optional mindestens eine sterisch voluminöse Komponente, die innerhalb des Latexpolymers eingeschlossen ist;
mit der Maßgabe, dass die mindestens eine Wirkkomponente keine bioaktive Komponente ist.

8. Aktiver anionischer Polymerlatex nach Anspruch 7, worin die Zusammensetzung einen Film bilden kann.

9. Aktiver anionischer Polymerlatex nach Anspruch 8, worin der Film die Freisetzung der mindestens einen Wirkkomponente kontrolliert.

10. Aktiver anionischer Polymerlatex nach Anspruch 7, worin das mindestens eine ethylenisch ungesättigte erste Monomer eine Kombination aus Styrol und Butylacrylat ist.

11. Aktiver anionischer Polymerlatex nach Anspruch 7, worin das mindestens eine ethylenisch ungesättigte zweite Monomer Dimethylaminoethylmethacrylat oder Methacrylsäure oder Methoxypolyethylenglycolmethacrylat ist.

12. Aktiver anionischer Polymerlatex nach Anspruch 7, worin mindestens ein Teil der mindestens einen Wirkkomponente als Dispersion zur Latexzusammensetzung nachträglich zugegeben wird.

## Revendications

1. Procédé de fabrication d'un latex polymère anionique actif comprenant l'étape consistant à initier une polymérisation en émulsion d'une composition aqueuse comprenant, à tout moment pendant la polymérisation en émulsion :
a) au moins un premier monomère à insaturation éthylénique ;
b) éventuellement, au moins un deuxième monomère à insaturation éthylénique qui est anionique ou un précurseur à un anion ;
c) au moins un tensioactif anionique ;
d) au moins un composant actif ;
e) au moins un initiateur de radicaux libres ;
f) éventuellement, au moins un troisième monomère à insaturation éthylénique et à encombrement stérique ;
g) éventuellement, au moins un polymère à encombrement stérique ; et
h) éventuellement, au moins un tensioactif non-ionique,
à la condition que l'au moins un composant actif ne soit pas un composant bioactif.

2. Procédé de fabrication d'un latex polymère anionique actif selon la revendication 1, dans lequel le procédé est un processus semi-continu, et dans lequel au moins un composant actif est dissous dans la charge de monomères à tout moment pendant la polymérisation en émulsion.

3. Procédé de fabrication d'un latex polymère anionique actif selon la revendication 1, dans lequel le procédé est un processus discontinu, et dans lequel l'au moins un composant actif est présent à l'étape de semence de la polymérisation en émulsion.

4. Procédé de fabrication d'un latex polymère anionique actif selon la revendication 1, dans lequel l'au moins un premier monomère à insaturation éthylénique est du styrène, du styrène para-méthylique, du styrène chloro-méthylique, du vinyltoluène, de l'éthylène, du butadiène, du (méth)acrylate de méthyle, du (méth)acrylate d'éthyle, du (méth)acrylate de propyle, du (méth)acrylate de butyle, du (méth)acrylate de pentyle, du (méth)acrylate de glycidyle, du (méth)acrylate d'isodécyle, du (méth)acrylate de lauryle, du (méth)acrylonitrile, du (méth)acrylamide, du (méth)acrylamide de N-méthylol, du N-(isobutoxyméthyle)(méth)acrylamide, du néodécanoate de vinyle, du versatate de vinyle, de l'acétate de vinyle, un éther de vinyle alkyle en C₃ - C₈, un éther de vinyle alkoxy en C₃ - C₈, du chlorure de vinyle, du chlorure de vinylidène, du fluorure de vinyle, du fluorure de vinylidène, du trifluoroéthylène, du tétrafluoroéthylène, du chlorotrifluoroéthylène, de l'hexafluoropropylène, du chlorotrifluoroéthylène, de l'éthylène de perfluorobutyle, une alpha-oléfine en C₃ - C₈ perfluorée, un éther de vinyle alkyle en C₃ - C₈ fluoré, un éther de vinyle alkyle en C₃ - C₈ perfluoré, un éther de vinyle alkoxy en C₃ - C₈ perfluoré, ou une combinaison de ceux-ci.

5. Procédé de fabrication d'un latex polymère anionique actif selon la revendication 1, dans lequel l'au moins un deuxième monomère à insaturation éthylénique est de l'acide (méth)acrylique, de l'acide maléique, un anhydre maléique, du (méth)acrylate de 2-sulfoéthyle, du sulfonate de styrène, de l'acide sulfonique de 2-acrylamido-2-méthylpropane, du maléate de monométhyle, de l'acide itaconique, un anhydre itaconique, de l'acide fumarique, du méthacrylate de diméthylaminoéthyle, du méthacrylate de méthoxypolyéthylèneglycol ou une combinaison de ceux-ci.

6. Procédé de fabrication d'un latex polymère anionique actif selon la revendication 1, le procédé comprenant les étapes consistant à :
a) fournir une composition aqueuse comprenant :
i) au moins un premier monomère à insaturation éthylénique ;
ii) éventuellement, au moins un deuxième monomère à insaturation éthylénique qui est anionique ou un précurseur à un anion ;
iii) au moins un tensioactif anionique ;
iv) éventuellement, au moins un troisième monomère à insaturation éthylénique et à encombrement stérique ;
v) au moins un initiateur de radicaux libres ; et
vi) éventuellement, au moins un tensioactif non-ionique ;
b) initier une polymérisation en émulsion de la composition ; et
c) ajouter au moins un composant actif à la composition pendant le processus de polymérisation en émulsion,
à la condition que l'au moins un composant actif ne soit pas un composant bioactif.

7. Latex polymère anionique actif comprenant :
a) un polymère de latex comprenant le produit de polymérisation de :
i) au moins un premier monomère à insaturation éthylénique ; et
ii) au moins un deuxième monomère à insaturation éthylénique qui est anionique ou un précurseur à un anion ;
b) au moins un composant actif encapsulé au moins partiellement à l'intérieur du polymère de latex ; et
c) éventuellement, au moins un composant à encombrement stérique incorporé à l'intérieur du polymère de latex,
à la condition que l'au moins un composant actif ne soit pas un composant bioactif.

8. Latex polymère anionique actif selon la revendication 7, dans lequel la composition est capable de former un film.

9. Latex polymère anionique actif selon la revendication 8, dans lequel le film contrôle la libération de l'au moins un composant actif.

10. Latex polymère anionique actif selon la revendication 7, dans lequel l'au moins un premier monomère à insaturation éthylénique est une combinaison de styrène et d'acrylate de butyle.

11. Latex polymère anionique actif selon la revendication 7, dans lequel l'au moins un deuxième monomère à insaturation éthylénique est du méthacrylate de diméthylaminoéthyle ou de l'acide méthacrylique ou du méthacrylate de méthoxypolyéthylèneglycol.

12. Latex polymère anionique actif selon la revendication 7, dans lequel au moins une portion de l'au moins un composant actif est ajouté ultérieurement à la composition de latex comme dispersion.
